# EUROPEAN PATENT APPLICATION

(11) **EP 4 659 706 A1**
(43) Date of publication of application: **10.12.2025**
(21) Application number: 25181214.5
(22) Date of filing: 05.06.2025
(51) Int. Cl.: A61F 2/24

(54) **TISSUE FIXATION DEVICE WITH FOLDING GRIPPING ELEMENTS FOR ENHANCED TISSUE PROTECTION**

(30) Priority: 05.06.2024 US 202463656244 P
(71) Applicant: Evalve, Inc., Santa Clara, CA 95054 (US)
(72) Inventor: Abunassar, Chad, Alameda, CA 94501 (US); Gonzales, Gabriel, Milpitas, CA 95035 (US)
(74) Representative: Boult Wade Tennant LLP

(57) **Abstract**

A fixation device includes a first distal element and a gripping device. The gripping device including a base section, a first bend feature, and a first proximal element. The first bend feature defining a first hinge axis. The first proximal element extending from the first bend feature and having a first section, a second section, and a first hinge disposed between the first section and the second section and defining a second hinge axis. The second section having a plurality of frictional elements extending therefrom and being rotatable about the second hinge axis between a first configuration and a second configuration.

## Description

### BACKGROUND

The cardiac cycle is divided into two phases-diastole and systole. Diastole is generally characterized by the muscular relaxation of the heart and the filling of its chambers with blood. On the other hand, systole is generally characterized by the muscular contraction of the ventricles which pumps blood from the ventricles to the arteries. During ventricular systole, ventricular pressure increases relative to atrial pressure resulting in the closure of the mitral valve and the tricuspid valve. The mitral valve separates the left atrium from the left ventricle, and the tricuspid valve separates the right atrium from the right ventricle. These valves operate as check valves preventing blood from flowing back into the atria during ventricular contraction. However, valvular insufficiency may appear in one or both of these valves which may result in a regurgitative flow back into the atrium across the effected valve. Such regurgitative flow can be in the form of mitral valve regurgitation ("MVR") and/or tricuspid valve regurgitation ("TVR"). Left untreated, MVR and TVR can lead to severe health consequences, such as progressive heart failure, cardiac arrythmias, pulmonary hypertension, stroke, and endocarditis, to name a few.

MVR and TVR can have a variety of etiologies which typically fall into the categories of degenerative (primary) and functional (secondary) regurgitation. Degenerative valve regurgitation principally occurs due to abnormalities or degeneration of the valve apparatus, such as the valve leaflets, valve annulus, chordae tendineae, and/or papillary muscles. One example of a degenerative valve condition is mitral valve prolapse. Functional valve regurgitation is often a secondary condition that arises from underlying heart conditions or diseases that affect the structure or function of the heart. Examples of conditions that can result in functional regurgitation include dilated cardiomyopathy, ischemic heart disease, pulmonary hypertension, and heart failure. Regardless of the underlying condition precipitating the regurgitative flow, the primary mechanism by which regurgitation occurs is the failure of the valve leaflets to properly and completely seal or coapt during systole which allows a jet of blood to flow back into the atrium between the effected leaflets.

Treatment options for MVR and TVR generally include Guideline-Directed Medical Therapy ("GDMT"), valve replacement, and valve repair. GDMT usually involves the administration of a combination of drugs that treat an underlying heart condition. Valve replacement and repair may include open-heart surgical options and catheter-based options. Catheter-based repair procedures are sometimes referred to as transcatheter edge-to-edge repair ("TEER").

### BRIEF SUMMARY OF THE DISCLOSURE

In a first aspect of the present disclosure, a fixation device may include a first distal element and a gripping device. The gripping device may include a base section, a first bend feature defining a first hinge axis, and a first proximal element extending from the first bend feature. The first proximal element may include a first section, a second section, and a first hinge disposed between the first section and the second section and may define a second hinge axis. The second section may have a plurality of frictional elements that may extend therefrom and may be rotatable about the second hinge axis between a first configuration and a second configuration.

Additionally, in the first configuration, the first section may be aligned with the second section along a longitudinal axis, and in the second configuration, the second section may be angled relative the first section. Also, in the second configuration, the plurality of frictional elements of the second section may point in a direction towards the first section. The second section may include a distal surface. The distal surface may face the first distal element when the proximal element is in the first configuration and may face the first section when in the second configuration.

Also, the first proximal element may include a lever connected to the first hinge. The lever may extend in a direction toward the first section and may be configured to couple to a proximal element line. The first section may include a recess, and the lever may be disposed within the recess when the proximal element is in the first configuration. Alternatively, the first section may include a proximal surface, and the lever may be disposed above the proximal surface when the first proximal element is in the first configuration.

Further, the first section may include a plurality of frictional elements. The first proximal element may have an un-tensioned state and a tensioned state. The first proximal element may be rotatable about the first hinge axis between the un-tensioned state and the tensioned state. The first proximal element may be configured to be in the first configuration in the un-tensioned state and the tensioned state. The first proximal element may also have a high-tension state. The first proximal element may be in the high tensioned state when in the second configuration.

Additionally, the first proximal element may include a first elongate member, a second elongate member, and a second hinge. The first hinge may be on the first elongate member, and the second hinge may be on the second elongate member. The first proximal element may include a first end portion and a second end portion. The first end portion may be connected to the first bend feature, and the first and second elongate members may extend between the first and second end portions. The first and second elongate members may be offset from each other in a direction transverse to a longitudinal axis of the first proximal element so as to form a space therebetween. The first and second hinges may be aligned such that the first and second hinges together may define the second hinge axis. The first proximal element may include a lever connected to the second end portion and may extend into the space. The lever and first end portion may define an interface. The interface may be offset in a longitudinal direction relative to the second hinge axis.

Also, the fixation device may further include a center portion, and the base section may be connected to the center portion. The first distal element may also be connected to the center portion and may extend therefrom between a fixed end and a free end. The first distal element may have a tissue engagement surface extending between the fixed end and the free end. The base section may be connected to the first distal element.

In another aspect of the present disclosure, a fixation device may include a first distal element and a second distal element. The fixation device may also include a first proximal element disposed in opposition to the first distal element and may be rotatable about a first hinge axis relative to the first distal element. The first proximal element may have a first section, a second section, and a hinge disposed between the first and second sections. The hinge may define a second hinge axis. The second section may be rotatable about the second hinge axis toward the first section. The second section may have a plurality of frictional elements extending therefrom. The fixation device may further include a second proximal element disposed in opposition to the second distal element and rotatable about a first hinge axis relative to the second distal element. The second proximal element may have a first section, a second section, and a hinge disposed between the first and second sections. The hinge may define a second hinge axis. The second section of the second proximal element may be rotatable about the second hinge axis toward the first section of the second proximal element. The second section of the second proximal element may have a plurality of frictional elements extending therefrom.

Additionally, the first and second proximal elements may each include a lever connected to the hinge of the respective first and second proximal elements. The lever of each of the first and second proximal elements may include an opening extending therein. The opening may be configured to receive a proximal element line. The first section of each of the first and second proximal elements may include a plurality of frictional elements extending therefrom.

In further aspect of the present disclosure, a method for fixing leaflets of a heart valve may include positioning a fixation device adjacent the heart valve and moving a first and second distal element to an open position. The method may also include maintaining a first tension on a first proximal element line and a second proximal element line so that a first proximal element coupled to the first proximal element line is in a raised position relative to the first distal element and a second proximal element coupled to the second proximal element line is in a raised position relative to the second distal element. The first and second proximal elements may each include a hinge dividing the first and second proximal elements between a first section and a second section. The method may further include applying a second tension on the first and second proximal element lines greater than the first tension such that the second section of each of the first and second proximal elements rotates about the hinge toward the first section of each of the first and second proximal elements. The second section of each of the first and second proximal elements may have a plurality of frictional elements extending therefrom.

### BRIEF DESCRIPTION OF THE DRAWINGS

FIG. 1A is a cross-sectional representation of a heart illustrating its four valves.
FIG. 1B is a cross-sectional representation of a heart illustrating the left ventricle and left atrium during systole.
FIG. 2A is a schematic view of a mitral valve during normal coaptation.
FIG. 2B is a schematic view of a mitral valve during regurgitate coaptation.
FIGS. 3A and 3B are schematic views of a fixation device according to an embodiment of the present disclosure grasping leaflets of a mitral valve.
FIG. 4A is a perspective view of a fixation device according to another embodiment of the present disclosure.
FIG. 4B is a perspective view of the fixation device of FIG. 4A including a covering.
FIG. 5A is a perspective view of a gripping device of the of the fixation device of FIG. 4A according to an embodiment of the present disclosure.
FIG. 5B is an elevational view of the gripping device of FIG. 5A.
FIG. 6A is a perspective view of a gripping device according to another embodiment of the present disclosure.
FIG. 6B is a partial schematic view of the gripping device of FIG. 6A coupled to a distal element of the fixation device of FIG. 4A.
FIG. 6C is a partial schematic view of a gripping device according to an alternative embodiment of the present disclosure coupled to a distal element according to an alternative embodiment of the present disclosure.
FIG. 7A is an elevational view of a coupling system according to an embodiment of the present disclosure for coupling the fixation device of FIG. 4A and a delivery system.
FIGS. 7B and 7C are schematic views of the coupling system of FIG. 7A in respective first and second configurations.
FIGS. 8A and 8B are schematic cross-sectional views of a coupling system according to another embodiment of the present disclosure for coupling a fixation device, such as the fixation device of FIG. 4A, and a delivery system.
FIGS. 9A-9B, 10A-10B, 11A-11B, 12A-12B and 13A-13C illustrate the fixation device of FIG. 4A in various possible positions during introduction and placement of the device within a mammalian body to perform a therapeutic procedure.
FIG. 14 is a perspective view of the fixation device of FIG. 4A including a locking mechanism according to an embodiment of the present disclosure and illustrating a plurality of proximal element lines and a lock line coupled to the fixation device.
FIG. 15 is an elevational view of the locking mechanism and proximal elements of the fixation device of FIG. 14 and illustrating a lock line and single proximal element line respectively coupled thereto.
FIG. 16 is a schematic view of the fixation device of FIG. 4A coupled to a delivery system and illustrating a plurality of proximal element lines coupled to a shaft of the delivery system.
FIGS. 17A and 17B are partial enlarged views of a distal end portion of the delivery system shaft of FIG. 16 according to an embodiment of the present disclosure.
FIG. 17C is a cross-sectional view of the delivery system shaft taken along line C-C of FIG. 17B.
FIG. 17D is a partial perspective view of a distal end portion of one of the proximal element lines of FIG. 16 including a catch element according to an embodiment of the present disclosure.
FIG. 17E is a partial elevational view of the delivery system shaft of FIG. 17A having holes configured to receive the catch element of FIG. 17D.
FIG. 17F is a partial elevational view of the delivery system shaft of FIG. 17A and an actuator rod disposed therein intersecting the holes of the delivery system shaft.
FIG. 17G is a partial elevational view of a distal end portion of one of the proximal element lines of FIG. 16 including a catch element according to another embodiment of the present disclosure.
FIG. 18A is an enlarged cross-sectional view of the locking mechanism of FIG. 14 taken along a midline thereof and in an unlocked configuration.
FIG. 18B is an enlarged elevational view of the locking mechanism of FIG. 14 and in a locked configuration.
FIG. 18C is a perspective view of a release harness of the locking mechanism of FIG. 14.
FIG. 19A is an elevational view of a locking mechanism of the fixation device of FIG. 4A according to another embodiment of the present disclosure.
FIG. 19B is a transparent perspective view of a binding plate of the locking mechanism of FIG. 19A.
FIG. 19C is an enlarged elevational view of the locking mechanism of FIG. 19A.
FIG. 20A is a perspective view of a gripping device according to another embodiment of the present disclosure.
FIG. 20B is a top view of the gripping device of claim 20A.
FIG. 20C is an elevational view of the gripping device of claim 20A.
FIG. 20D is partial schematic view of a proximal element of the gripping device of FIG. 20A in an exemplary first configuration.
FIG. 20E is a partial schematic view of the proximal element of FIG. 20D in an exemplary second configuration.
FIG. 21A is a perspective view of a gripping device according to a further embodiment of the present disclosure.
FIG. 21B is a top view of the gripping device of FIG. 21A.
FIG. 21C is an elevational view of the gripping device of FIG. 21A.
FIG. 22A is a perspective view of a gripping device according to another embodiment of the present disclosure.
FIG. 22B is a top view of the gripping device of FIG. 22A.
FIG. 22C is an elevational view of the gripping device of FIG. 22A.
FIG. 22D is a partial schematic view of a proximal element of the gripping device of FIG. 22A.
FIG. 22E is a partial schematic view of a proximal element of the gripping device of FIG. 22A according to another embodiment of the present disclosure.
FIG. 23A is a perspective view of a gripping device according to a further embodiment of the present disclosure and in an exemplary first configuration.
FIG. 23B is a perspective view of the gripping device of FIG. 23A in an exemplary second configuration.
FIG. 23C is a perspective view of the gripping device of FIG. 23A in an exemplary third configuration.
FIG. 24A is a perspective view of a delivery device according to an embodiment of the present disclosure.
FIG. 24B is a partial cross-sectional view of a proximal element actuator handle of the delivery device of FIG. 24A.
FIG. 25 is a partial elevational view of a delivery device according to another embodiment of the present disclosure.

### DETAILED DESCRIPTION

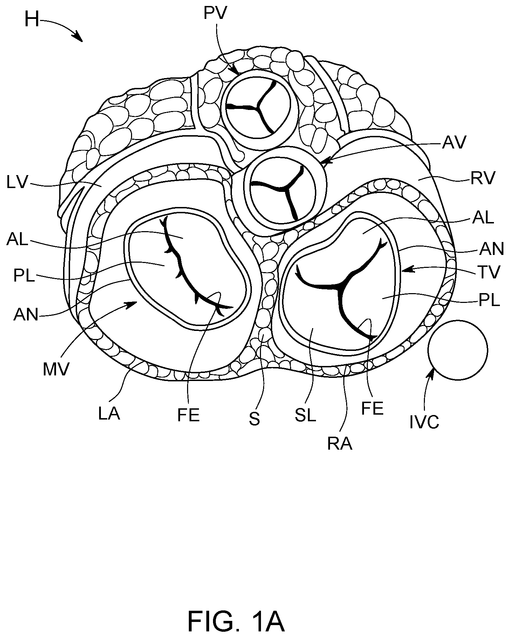

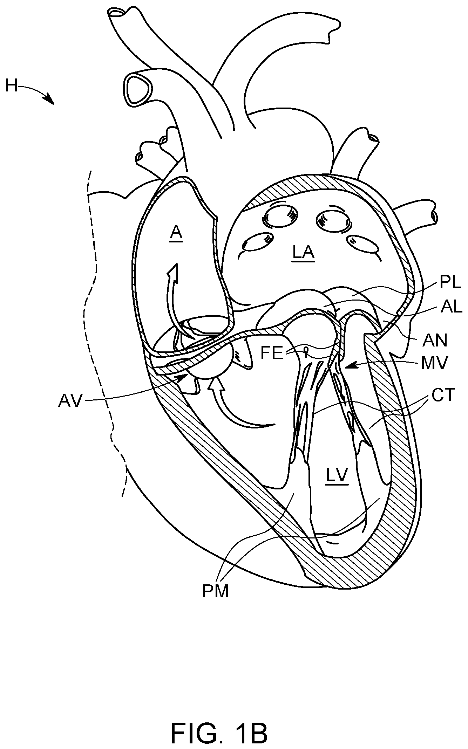

The valves of a normal heart H are illustrated in FIGS. 1A and 1B. These valves include the mitral valve MV, the tricuspid valve TV, the aortic valve AV, and the pulmonary valve PV. The mitral valve MV separates the left atrium LA and the left ventricle LV, and the tricuspid valve TV separates the right atrium RA and the right ventricle RV. The mitral valve MV and the tricuspid valve TV are sometimes referred to as the atrioventricular valves. The mitral valve MV is a bicuspid valve in that it has two leaflets referred to as the posterior leaflet PL and the anterior leaflet AL. The tricuspid valve TV typically has three leaflets referred to as the anterior leaflet AL, the posterior leaflet PL, and the septal leaflet SL. However, studies have shown that, although the TV is typically composed of three leaflets of unequal size, in many cases, two or more than three leaflets may be present as anatomic variants in healthy subjects. Thus, reference herein to the tricuspid valve TV should be understood to refer to the atrioventricular valve located between the right atrium RA and right ventricle RV regardless of the number of leaflets be it two, three, or more than three leaflets. However, exemplary embodiments discussed herein refer to the usual anatomic structure of the tricuspid valve TV that includes three leaflets.

As illustrated in FIG. 1B, the anterior leaflet AL and posterior leaflet PL of the mitral valve MV extend from a valve annulus AN to respective free edges FE. The free edges FE are secured to the lower portions of the left ventricle LV through chordae tendineae CT (referred to hereinafter as the chordae). The chordae CT include a plurality of branching tendons that are attached to papillary muscles PM at the lower portions of the left ventricle LV and extend upwardly to the lower surfaces of each of the valve leaflets where they are attached. The three leaflets of the tricuspid valve TV similarly extend from a valve annulus AN to respective free edges FE which are secured via chordae to the papillary muscles of the right ventricle RV.

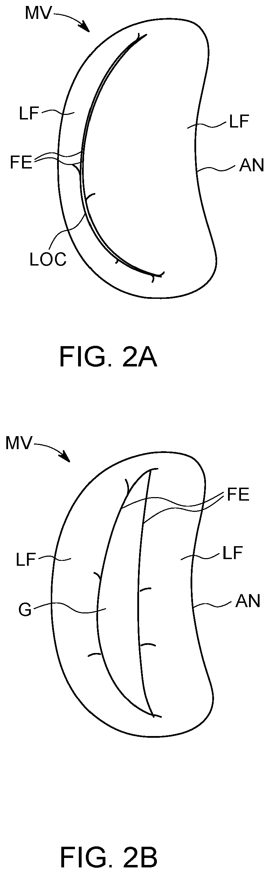

The mitral valve MV depicted in FIGS. 1B and 2A illustrate the proper functioning of an atrioventricular valve during ventricular systole. As the ventricles contract, the free edges FE of adjacent leaflets LF meet along a line of coaptation LOC. The joinder of the leaflets LF at this line of coaptation LOC seals off the ventricle from the atrium and prevents the back flow of blood or "regurgitation" from entering into the atrium. Thus, with the right atrium RA and left atrium LA respectively sealed off by the mitral valve MV and tricuspid valve TV, blood in the left ventricle LV can only flow through the aortic valve AV to the body, and blood in the right ventricle RV can only flow through the pulmonary valve PV to the lungs.

A number of structural defects in the heart H can cause mitral valve regurgitation ("MVR") and/or tricuspid valve regurgitation ("TVR"). MVR and TVR occur when their respective leaflets LF do not close properly allowing leakage from the ventricle into the atrium. The mitral valve MV depicted in FIG. 2B illustrates valvular insufficiency of an atrioventricular valve resulting in regurgitation. In the depicted example, an enlargement of the heart H may cause the valve annulus AN to become enlarged, making it impossible for the free edges FE of the valve leaflets LF to meet during systole. This may result in a gap G between the leaflets LF which allows blood to leak through the valve. In another example, ruptured or elongated chordae CT can cause a valve leaflet LF to prolapse at least due to inadequate tension transmitted to the leaflet via the chordae CT. While an adjacent leaflet LF may maintain a normal profile, the prolapsing leaflets LF may flail about preventing the proper joinder between the leaflets LF resulting in leakage into the atrium. In a further example, regurgitation can occur in patients who have suffered ischemic heart disease which may result in weak ventricular contractions insufficient to effect proper closure.

The present disclosure describes exemplary systems, devices, and methods for percutaneously repairing a valve to treat cardiac valve regurgitation, particularly MVR and TVR. When referring to such disclosed systems, devices, and methods, the term "proximal" (P) shall mean closer to the user or in a direction toward a device to be manipulated by the user outside the patient's body, and the term "distal" (D) shall mean more distant from the user or in a direction toward a device that is positioned at the treatment site within the patient's body (e.g., fixation device 112). With respect to the mitral valve and tricuspid valve, "proximal" shall refer to the atrial or upstream side of the valve leaflets, and "distal" shall refer to the ventricular or downstream side of the valve leaflets.

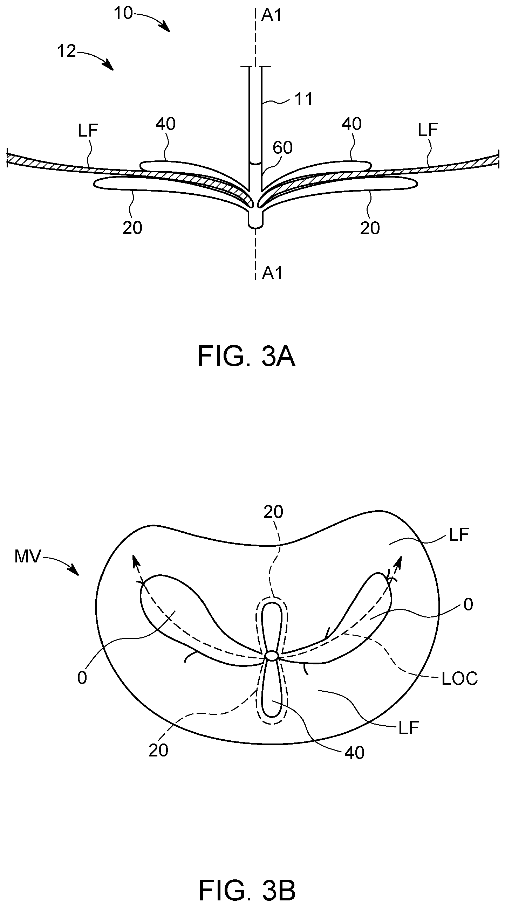

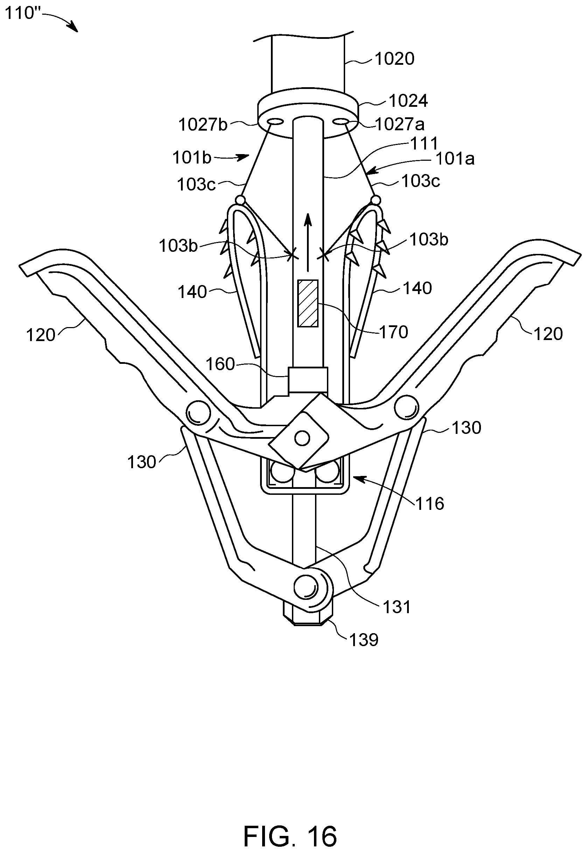

FIGS. 3A and 3B depict a fixation device 12, according to an embodiment of the present disclosure, grasping leaflets LF of an atrioventricular valve, which is illustrated as a mitral valve MV. Fixation device 12 may be releasably coupled to a distal end of a shaft 11 of a delivery system 600 (see FIG. 16) to form an interventional tool 10. Fixation device 12 may include distal elements 20 (also referred to herein as fixation elements) and proximal elements 40 (also referred to herein as gripping elements). Distal and proximal elements 20, 40 may be moveable relative to each other and may protrude radially outward relative to a longitudinal axis A1 of fixation device 12. As shown in FIG. 3A, fixation device 12 may be positionable on opposite sides of adjacent leaflets LF of the valve so as to capture or retain the leaflets LF therebetween. In this regard, proximal elements 40 may be positioned at a proximal side of the valve leaflets LF, and distal elements 20 may be positioned on a distal side of the valve leaflets LF. Proximal elements 40 may be made from cobalt chromium, nitinol, or stainless steel, for example, and distal elements 20 may be made from cobalt chromium or stainless steel, for example.

Fixation device 12 may be releasably coupled to shaft 11 such that it can be detached and left behind as an implant to hold the leaflets LF together in the coapted position. In this regard, fixation device 12 may be delivered to a target valve percutaneously using any one of a number of different approaches, such as via a transfemoral, a transapical, or a transjugular approach, for example. Thus, in one example of treating MVR, fixation device 12 may be delivered to the deficient mitral valve MV using a transfemoral approach in which fixation device 12 is guided through the inferior vena cava IVC (see FIG. 1A), across the interatrial septum S, and into left atrium LA where fixation device 12 is advanced into the mitral valve MV. Also, in one example of treating TVR, fixation device 12 may be guided transfemorally through the inferior vena cava IVC to the right atrium RA where fixation device 12 is advanced to a desired position within the tricuspid valve TV.

FIG. 3B is an atrial-side view of fixation device 12 in one example of a desired orientation in relation to adjacent leaflets LF of an atrioventricular valve, such as the depicted mitral valve MV. The distal and proximal elements 20, 40 are positioned to be substantially perpendicular to the line of coaptation LOC. Thus, in the case of a mitral valve MV, fixation device 12 may be oriented perpendicular (+/- 5 degrees) to a line of coaptation LOC between the posterior leaflet PL and anterior leaflet AL, and in the case of a tricuspid valve TV, fixation device 12 may be positioned perpendicular (+/- 5 degrees) to a line of coaptation between the septal leaflet SL and the anterior leaflet AL, the septal leaflet SL and the posterior leaflet PL, or the anterior leaflet AL and the posterior leaflet PL, for example. Device 12 may be moved roughly along the line of coaptation LOC to the location of regurgitation. The leaflets LF may be held in place so that, during diastole, the leaflets LF remain in position between elements 20, 40 surrounded by openings O (also referred to herein as orifices) which result from the diastolic pressure gradient. Advantageously, leaflets LF are coapted such that their proximal or upstream surfaces face each other in a vertical orientation, parallel to the direction of blood flow through the valve. The upstream surfaces may be brought together so as to be in contact with one another or may be held slightly apart but will preferably be maintained in the vertical orientation in which the upstream surfaces face each other at the point of coaptation. This simulates the double orifice geometry of a standard surgical bow-tie repair. Color Doppler echo will show if the regurgitation of the valve has been reduced. If the resulting flow pattern is satisfactory, the leaflets LF may be fixed together in this orientation. If the resulting color Doppler image shows insufficient improvement in valve regurgitation, fixation device 112 may be repositioned. This may be repeated until an optimal result is produced wherein the leaflets LF are held in place.

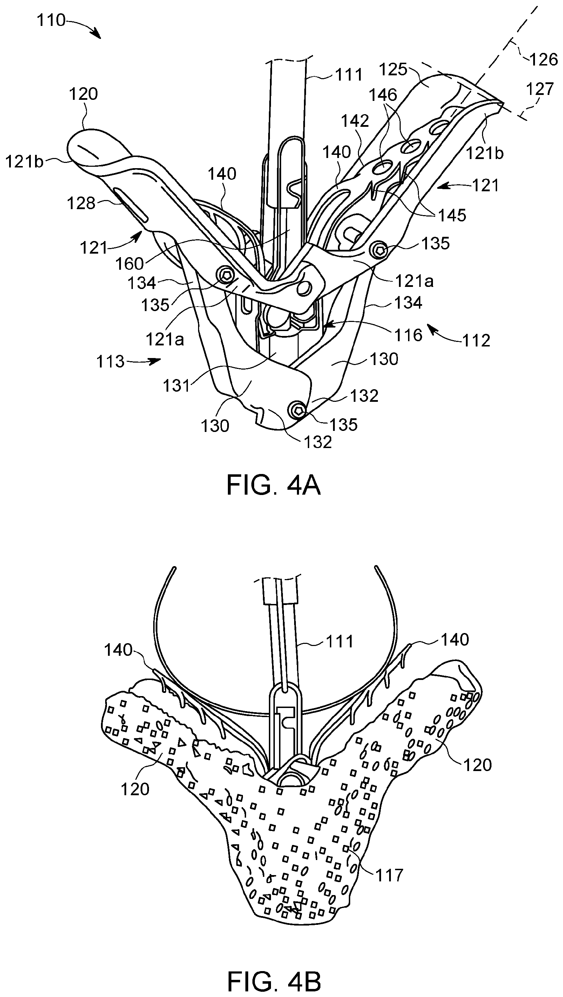

FIGS. 4A-19C depict a fixation device 112 according to another embodiment of the present disclosure. Fixation device 112 may generally include a pair of distal elements 120, a pair of proximal elements 140, a coupling member 160, an actuator 113, and a stud 131. Distal elements 120 may include elongate arms 121 in which each arm has a proximal end portion 121a, which may be rotatably connected to the coupling member 160, and a free end 121b, as best shown in FIG. 4A. Free ends 121b may each have a rounded shape to minimize interference with and trauma to surrounding tissue structures according to one example. In one example, each free end 121b defines a curvature extending about two axes 126, 127. The first axis 126 may be a longitudinal axis of each respective arm 121. Additionally, arms 121 may each include an engagement surface 125 that may also be curved about first axis 126 and may extend at least partially along a length of arm 121 to the free end 121b. Thus, in some examples, engagement surfaces 125 may each have a cupped or concave shape which may maximize contact area engagement with tissue and may assist in grasping and holding valve leaflets. Such cupped or concave shape may further allow arms 121 to nest around shaft 111 of interventional tool 110 while in the closed position to minimize the profile of device 112. Thus, arms 121 may be at least partially cupped or curved inwardly about their longitudinal axes 126 which may form a concavity extending along axis 126 which may nest proximal elements 140 when in a lowered position thereof. The second axis 127 about which each free end 121b may be curved may extend perpendicular to first axis 126, as is also shown in FIG. 4A. The curvature about this second axis 127 may be a reverse curvature located at the most distal portion of free ends 121b. In addition to the dual curvature, free ends 121b may flare outwardly at their respective longitudinal edges. It is believed that both the reverse curvature and flare help create an atraumatic configuration that minimizes trauma to the tissue engaged therewith.

In the nonlimiting embodiment depicted, a transverse width across engagement surfaces 125 (which is in the direction of second axis 127 and determines the width of tissue engaged) may be at least about 2 mm, 3-10 mm in some examples, and about 4-6 mm in some examples. In some embodiments, a wider engagement may be desired wherein the engagement surfaces 125 are larger, for example about 2 cm, or multiple fixation devices 112 may be used adjacent to each other. Arms 121 may also have a length of about 6-12 mm (defined along first axis 126), and engagement surfaces 125 may be configured to engage a length of tissue of about 4-10 mm along the longitudinal axis 126 of arms 121 according to some examples. Also, as shown in the illustrated example, each arm 121 may include a plurality of openings 128 to enhance grip and to promote tissue ingrowth following implantation.

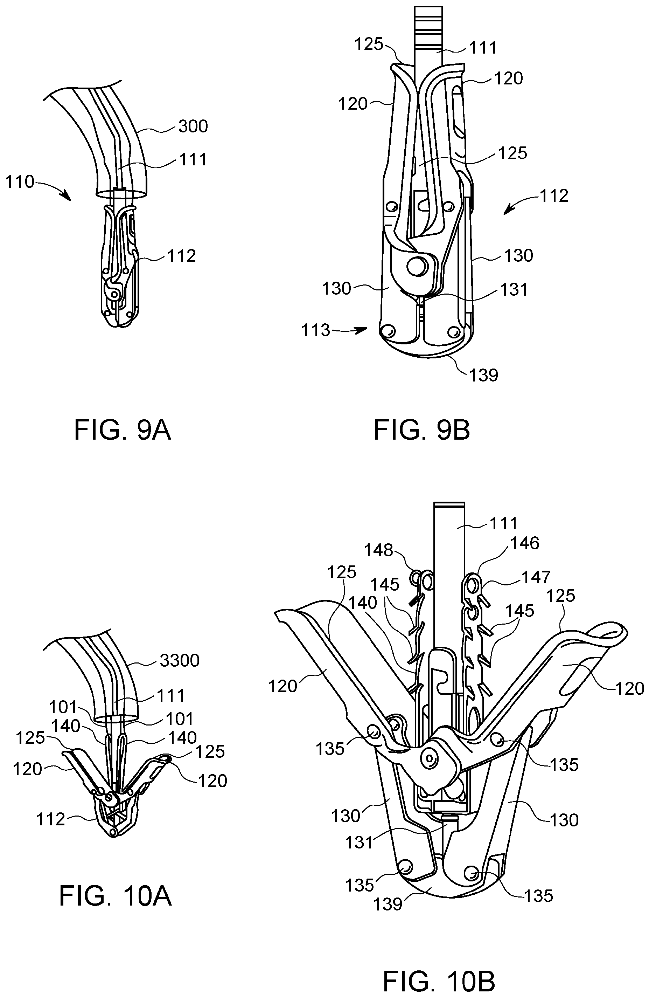

In one example, actuator 113 may include two link members or legs 130. Legs 130 may be comprised of a rigid or semi-rigid metal or polymer such as Elgiloy^{®}, cobalt chromium or stainless steel, however any suitable material may be used. Each leg 130 may have a first end 132, which may be rotatably joined with one of the distal elements 120 at a riveted joint 135, and a second end 134, which may be rotatably joined with stud 131, as shown in FIG. 4A. Although the depicted embodiment shows both legs 130 pinned to stud 131 by a single rivet 135, it is also contemplated that each leg 130 may be individually attached to the stud 131 by a separate rivet, pin or the like. In other embodiments of actuator 113, actuator 113 may include a base 139, and second ends 134 of legs 130 may be rotatably joined with base 169, such as by one or more riveted joints 135, as best shown in FIG. 10B. An actuator rod 170 of delivery 600 may be joinable with actuator 113 directly, such as via direct connection with base 139, or indirectly, such as via connection with stud 131, which itself may extend from base 139. In either of these embodiments, actuator rod 170 may be axially extendable and retractable in a proximal-distal direction to actuate actuator 113 and consequently rotate distal elements 120 between open, closed, and inverted positions, which are described further below. Additionally, coupling member 160, stud 131, and/or base 169 may comprise a center portion or center body of fixation device, for example.

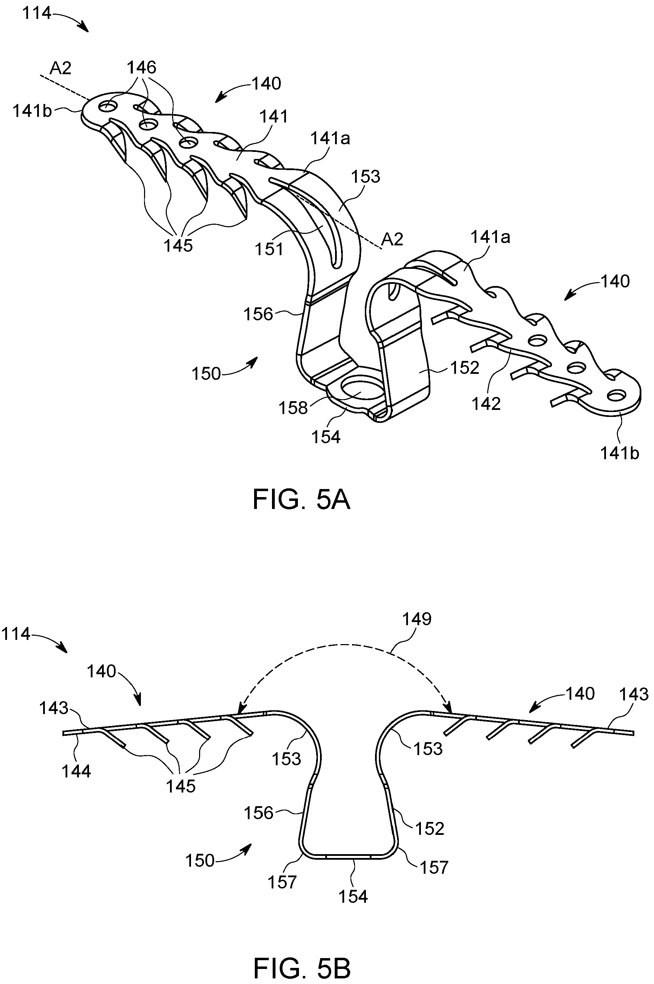

Proximal elements 140 may, in some examples, be flexible, resilient, and cantilevered from a center of fixation device 112. For example, FIGS. 5A and 5B depict a gripping device 114 according to an embodiment of the present disclosure that may generally include a pair of proximal elements 140, a base section 150, and a pair of arm bend features 153 partitioning proximal elements 140 from base section 150.

Proximal elements 140 may be in the form of elongate arms 141 that each extend along a longitudinal axis A2 from a first end portion or fixed end 141a to a second end portion or free end 141b, as shown in FIG. 5A. Each proximal element 140 may also have opposed side edges 142 that define a width transverse to the longitudinal axis A2. Such width may be less than the width of a corresponding distal element 120 such that proximal element 140 may be recessed within the concavity formed by engagement surface 125 of distal element 120 when proximal element 140 is moved into a lowered position, as described in more detail below.

Proximal elements 140 may also each have a first side or proximal side 143 and a second side or distal side 144. In one example, proximal elements 140 may include a plurality of openings 146 that may extend from proximal side 143 to distal side 144, as shown in FIG. 5A. Such openings 146 may be used to couple a proximal element line, which is discussed further below, to a proximal element 140 for raising and lowering proximal element 140. Each proximal element 140 may also include one or more frictional elements 145 extending from distal side 144. For example, each proximal element 140 may include one or more rows of frictional elements 145 where frictional elements 145 in each row may be aligned in a direction transverse to longitudinal axis A2. Frictional elements 145 in such rows may also be aligned with frictional elements 145 in other rows in a lengthwise direction thereby forming columns of frictional elements 145. For example, in the embodiment depicted in FIGS. 5A and 5B, each proximal element 145 may include four rows of two frictional elements 145. In other words, two columns of four frictional elements 145. In other embodiments, proximal elements 140 may include one to six rows of two to six frictional elements 145 per row, for example. However, in other embodiments, frictional elements 145 may be arranged in an offset relationship in a lengthwise and/or transverse direction such that at least some frictional elements 145 are not aligned with another frictional element 145 in such directions.

Frictional elements 145 may comprise frictional protrusions or tines having tapering pointed tips extending from distal side 144 of proximal elements 140. Frictional elements 145 may also be angled toward fixed end 141a of proximal element 140 which may help prevent frictional elements 145 from inadvertently snaring tissue during repositioning of fixation device 112. In one example, frictional elements 145 may be integral with or connected to a distal surface 144 of a proximal element 140 and protrude therefrom. In another example, as shown in FIG. 5A, frictional elements 145 may be formed from side edges 142, such as by cutting and bending the base material forming proximal elements 140, for example. It may be appreciated that any suitable frictional elements may be used, such as prongs, windings, bands, barbs, grooves, channels, bumps, surface roughening, sintering, high-friction pads, coverings, coatings, or a combination of these. However, it should be noted that some types of frictional elements that can be utilized may permanently alter or cause some trauma to the tissue engaged. Thus, it is preferable that frictional elements 145 be atraumatic and generally frictional rather than penetrative so as to not injure or otherwise affect the tissue in a clinically significant way.

Base section 150 may be connected to a center portion or center body of fixation device 112 such that proximal elements 140 extend outwardly therefrom. For example, base section 150 may be coupled to coupling member 160. In the embodiment depicted, base section 150 may include a first member 152, a second member 154, and a third member 156. First and third members 152, 156 may be connected to second member 154 to form a generally U-shaped or box-shaped structure which may allow a lock (discussed below) to be positioned between first and third members 152, 156. However, other shapes may be formed, such as a V-shape, a crescent shape, or semicircular, for example. In some embodiments, first and third members 152, 156 may be connected to second member 154 via base bend features 157, for example. Also, second member 154 may include an opening 158 extending therethrough for receipt of stud 131 and/or actuator rod 170, as shown in FIG. 5A.

Arm bend features 153 may couple a respective proximal element 140 and base section 150. For example, an arm bend feature 153 can couple a proximal element 140 to first member 152 of base section 150, and another arm bend feature 153 can coupled the other proximal element 140 to third member 156 of base section 150. As shown, arm bend features 153 may form a living hinge about which proximal elements 140 may bend relative to base section 150. In this regard, arm bend features 153 may be integral with proximal elements 140 and base section 150 and may bias proximal elements 140 to a relaxed position. As illustrated in FIG. 5B, proximal elements 140 may form a relaxed angle 149 formed between proximal sides 143 of each proximal element 140. Such relaxed angle 149 is formed when proximal elements 140 are in the relaxed position and may form an angle of about 85 degrees to 200 degrees (+/- 5 degrees). For example, proximal elements 140 may form a relaxed angle of 180 degrees in the relaxed position. In another example, proximal elements 140 may form a relaxed angle of 185 degrees in the relaxed position. Although the embodiment depicted illustrates bend features 153 as living hinges, in other embodiments bend features 153 may comprise a biased hinge that modularly connects proximal elements 140 to base section 150. For example, proximal elements 140 may be separately formed from base section 150 and modularly connected to base section 150 via arm bend features 153 which may each comprise a spring biased hinge biasing a respective proximal element 140 to the relaxed position, for example.

Arm bend features 153 may also each include an elongate opening extending 151 along the longitudinal axis A2 which may furcate each arm bend feature 153, as illustrated in FIG. 5A. Such an elongated opening 151 may have a uniform width extending along axis A2. However, in some embodiments, such as the embodiment depicted, elongate opening 151 may form a bowling-pin shape such that a width of opening 151 is narrower at one end (e.g., the end closest to free end 141b) than the other end (e.g., the end furthers from free end 141b) and is wider somewhere in between. Elongate opening 151 may also not be relegated to just arm bend feature 153 but may also extend from arm bend feature 153 to proximal element 140 and/or base body 150. The elongate opening 151 and corresponding furcation of arm bend features 153 may be configured (e.g., in size, shape, spacing, position, etc.) so as to provide the desired resiliency, fatigue resistance, and/or flexibility at the coinciding arm bend features 153.

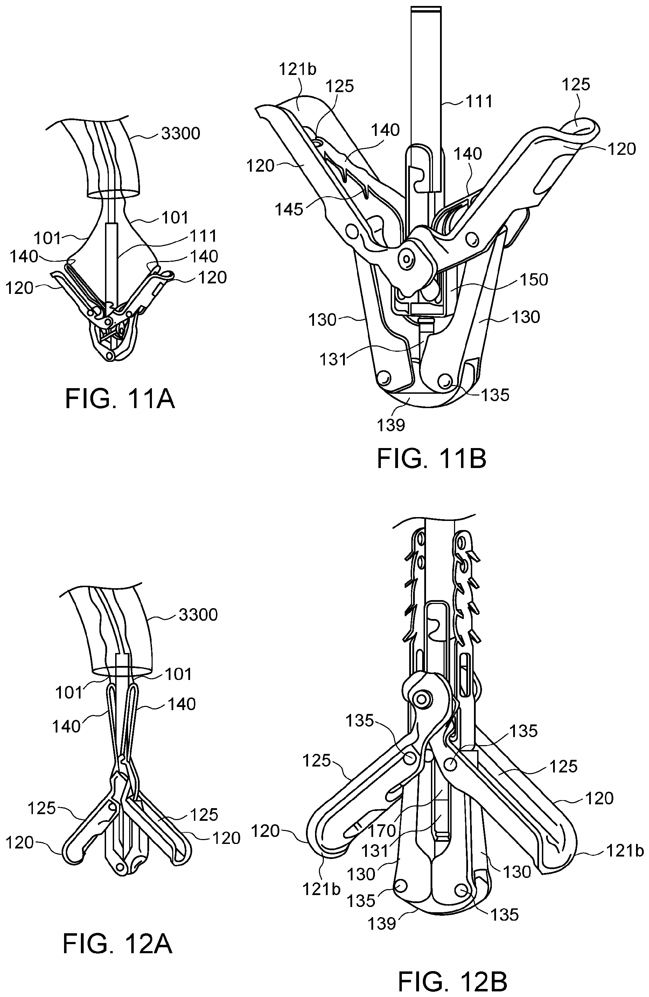

Base bend features 110 and arm bend features 112 may be configured to give gripping device 116 a bent configuration when gripping device is in a relaxed state (i.e., when proximal elements are in the relaxed position), such that when gripping device 114 is forced into a stressed state (e.g., by bending proximal elements at one or more of the base and/or arm In the exemplary embodiment depicted, gripping device 114 may be formed from a metallic sheet of a spring-like material, such as a shape-memory metal (e.g., Nitinol) which may provide the bias of proximal elements 140 toward the relaxed position. Alternatively, gripping device 114 could be molded from a biocompatible polymer. Each proximal element 112 may, in one example, be configured to be at least partially recessed within the concavity of the distal element 120 when no tissue is present. When fixation device 112 is in the open position, each proximal element 140 may be separated from the engagement surface 125 near free end 121b of arm 121 and may slope toward engagement surface 125 near free end 121b with the free end 141b of proximal element 140 contacting engagement surface 125, as illustrated in FIGS. 4A and 11B. This arrangement may be facilitated by the dimensions of base section 150. For example, increasing or decreasing the respective lengths of first, second, and third members 152, 154, 156 of base section 150 may increase or decrease the separation distance between a proximal element 140 and corresponding distal element 120 which may help accommodate a valve leaflet or other tissues of varying thicknesses. Further examples of gripping devices that may be utilized in fixation device 112 are described in more detail in U.S. Patent No. 11,096,691, the disclosure of which is incorporated by reference herein in its entirety.

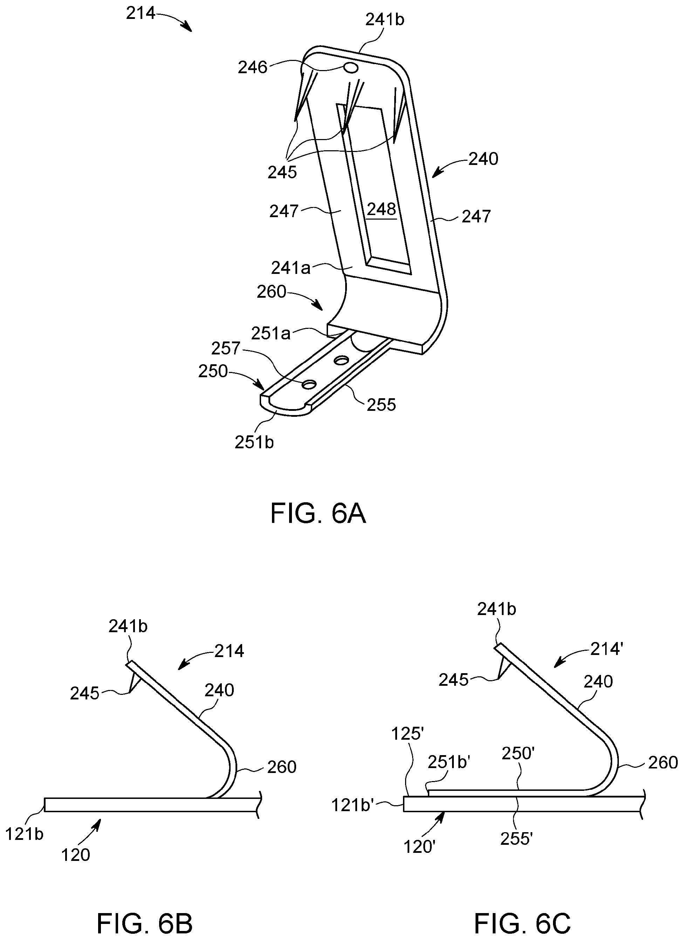

In other embodiments proximal elements may be connected to or otherwise extend from distal elements rather than from a center of fixation device, like that of fixation device 112. For example, FIGS. 6A and 6B depict a gripping device 214 according to another embodiment of the present disclosure that may generally include a first arm 240, a second arm 250, and an arm bend feature 260 partitioning first arm 240 from second arm 250. Gripping device 214 may be made from a shape-memory-metal material, such as Nitinol, for example.

First arm 240 may constitute a proximal element of fixation device 112, like that of and as an alternative to proximal element 140 and may include one or more frictional elements 245 which may be similar to frictional elements 145 discussed above. Thus, a plurality of frictional elements 245 may extend from a distal side of first arm 240 such as in one or more rows and/or columns. In the embodiment depicted, a single row of three frictional elements 245 may be provided near a free end 241b of first arm 240. But, as mentioned above, first arm 240 may have any number of frictional elements 245, such as two, four, or six, for example. First arm 240 may also include a pair of elongate members 247 offset from each other to form a space 248 therebetween. Such space 248 may be configured to receive second arm 250, for example. Additionally, first arm 240 may include one or more openings 246, such as near free end 241b, as shown in FIG. 6A. Such opening 246 may be configured to receive a proximal element line for raising and lowering first arm 240.

Second arm 250 may be in the form of a beam or other elongate structure. Second arm 250 (also referred to herein as base section) may be configured to couple to a distal element 120. For example, in the embodiment depicted in FIGS. 6A and 6B, second arm 250 may be curved in a plane transverse to its longitudinal axis. For example, second arm 250 may be semi-cylindrical such that it may have a semi-circular profile. Thus, second arm 250 may have a convex surface 255 configured to conform to the cupped curvature of engagement surface 125 of a corresponding distal element 120. FIG. 6B illustrates second arm 250 coupled to proximal engagement surface 125 of distal element 120 such that it is generally recessed within distal element 120 and free ends 241b, 251b of first and second arms 240, 250 point in the general direction toward free end 121b of distal element 120. Thus, in some embodiments, second arm 250 may have a width configured to be positioned within the concavity of distal element 120 and secure to proximal engagement surface 125. In other embodiments, a second arm 250' of an alternative gripping device 214' may not be concave and may instead have a planar surface corresponding to a planar engagement surface 125' of an alternative distal element 120' and secured thereto, as illustrated in FIG. 6C. In further embodiments, distal element 120 may include a recess or pocket for receipt and securement of second arm 250, such as in a press-fit manner, for example. Second arm 250 may be secured to distal element 120 in any number of ways, such as via one or more sutures, welding, press-fit, fastener (e.g., rivet or screw) or the like. For example, a rivet, screw, or suture may pass through one or more openings 257 in second arm 250 and into distal element 120. A tissue fixation device, such tissue fixation device 112, may include a pair of gripping devices 214 with one coupled to each distal element 120 as mentioned above.

Arm bend feature 260 may be coupled to a fixed end 241 of first arm 240 and a fixed end 251a of second arm 250 such that first and second arms 240, 250 extend in the same general direction and may form a V-shape when first arm 240 is in an exemplary open or raised position, as illustrated in FIGS. 6B and 6C. As shown, arm bend feature 260 may form a living hinge about which first arm 240 may bend relative to second arm 250. In this regard, arm bend feature 260 may be integral with first arm 240 and second arm 250 so as to form a monolithic structure and may bias first arm 240 to a relaxed position. Such relaxed position may include second arm 250 extending through space 248 between elongate members 247 of first arm 240 to form an X-shape. However, it should be noted that such position can generally only be achieved when gripping device 214 is not coupled to distal element 120 as the presence of distal element 120 would prevent second arm 250 from passing into space 248. It should also be appreciated that in some embodiments of gripping device 214, arm bend feature 260 may be a spring loaded or otherwise biased hinge coupling separately formed first and second arms 240, 250.

Fixation device 114 may also have a covering 117, as shown in FIG. 4B. As depicted, covering 117 may encapsulate distal elements 120 and actuator 113. Thus, engagement surfaces 125 may be covered by covering 117 which may help minimize trauma on tissues and enhance primary fixation via additional friction to assist in grasping. Additionally, covering 117 on engagement surfaces 125 may facilitate tissue ingrowth to provide for secondary fixation to ensure long-term security. Covering 117 may be loosely fitted and/or may be flexible such that device 112 can freely move to various positions all the while covering 117 conforms to the contours of the device 112 and remains securely attached thereto. It may be appreciated that the covering 117 may cover specific parts of fixation device 112 while leaving other parts exposed. For example, proximal elements 140 may be exposed, while distal elements 120 and actuator 113 may be covered. However, in some embodiments, proximal elements 140 may be covered with covering 117 to enhance grip and tissue ingrowth following implantation. Preferably, when a covering 117 is used in combination with frictional elements 145 or other frictional features, such as those extending from proximal elements 140, such features may protrude through such covering 117 so as to contact any tissue engaged by proximal elements 140.

Covering 117 may be comprised of any biocompatible material, such as polyethylene terepthalate, polyester, cotton, polyurethane, expanded polytetrafluoroethylene (ePTFE), silicon, or various polymers or fibers and have any suitable form, such as a fabric (woven or unwoven), mesh, textured weave, felt, looped or porous structure. Generally, covering 117 has a low profile so as not to interfere with delivery through an introducer sheath or with grasping and coapting of leaflets or tissue. Covering 117 may alternatively be comprised of a polymer or other suitable materials dipped, sprayed, coated, or otherwise adhered to the surfaces of the fixation device 112. Optionally, a polymer coating may include pores or contours to assist in grasping the tissue and/or to promote tissue ingrowth. Any of the coverings 117 may optionally include drugs, antibiotics, anti-thrombosis agents, or anti-platelet agents such as heparin, COUMADIN^{®} (Warfarin Sodium), to name a few. These agents may, for example, be impregnated in or coated on the coverings 117. These agents may then be delivered to the grasped tissues surrounding tissues and/or bloodstream for therapeutic effects.

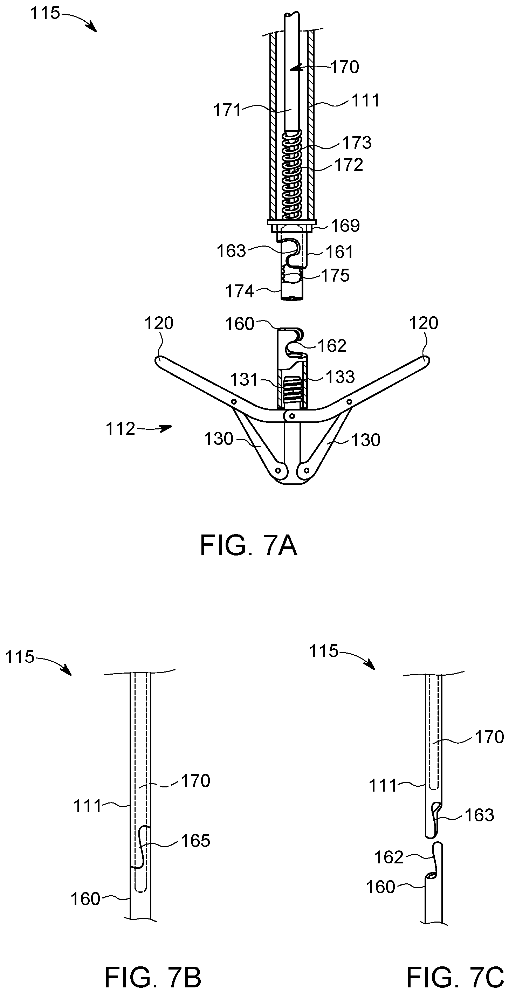

FIGS. 7A-7C depict an exemplary coupling system 115 between fixation device 112 and delivery system shaft 111. As mentioned above, once the leaflets of a target valve are coapted in the desired arrangement, fixation device 112 may then be detached from delivery 600 and left behind as an implant to hold the leaflets together in the coapted position. Such detachment may occur between coupling member 160 of fixation device 112 and a distal end of delivery shaft 111. Thus, coupling member 160 may be configured to be releasably coupled to shaft 111. Coupling member 160 may be disposed at a center of fixation device 112 and may extend proximally along it's the longitudinal axis of fixation device 112. In the coupling system 115 depicted, shaft 111 may form a tubular upper shaft with a first mating surface 163 formed at a distal end thereof, and coupling member 160 may form a detachable lower tubular shaft with a second mating surface 162 formed at a proximal end thereof. Mating surfaces 162, 163 may be correspondingly shaped so that they interlock and form a joining line 165 when merged together, as shown in FIG. 7B. In this regard, mating surfaces 162, 163 may have any shape or curvature which allows or facilitates interlocking and later detachment. For example, in the depicted embodiment, mating surfaces 162, 163 define a joining line 165 with an S-shaped curvature.

Coupling system 115 may also include actuator rod 170 and stud 131 (or alternatively base 139) such that fixation device 112 may also be releasably coupled to delivery 600 via connection between actuator rod 170 and stud 131. When shaft 111 is coupled to coupling member 160, they may collectively form an axial channel. Actuator rod 170 may pass through this channel to bridge the joining line 165, as shown in FIG. 7B. Actuator rod 170 may comprise a proximal extremity 171, a distal extremity 172, and a joiner 174. Distal extremity 172 may be smaller in diameter than proximal extremity 171 and may be optionally surrounded by a coil 173 which may serve to bias joiner 174 in a proximal direction. However, in some embodiments, actuator rod 170 may not have coil 173 or proximal and distal extremities 171, 172 of differing diameters. Joiner 174 may be removably coupled with stud 131 of fixation device 112 via any one of various possible release mechanisms. For example, in the embodiment depicted, joiner 174 may be threadedly connected to stud 131 of fixation device 112. In this regard, joiner 174 may have internal threads 175 which mate with external threads 133 on stud 131. Alternatively, joiner 174 may have external threads which mate with internal threads of stud 131. As described previously, stud 131 may be connected with distal elements 120 so that advancement and retraction of stud 131, by means of actuator rod 170, manipulates distal elements 120. It is also contemplated that joiner 174 may be directly threadedly engaged with base 139 where no stud 131 is provided. Once detachment of fixation device 112 is desired, actuator rod 170 may be rotated until threads 175 of joiner 174 disengage threads 133 of stud 131. Actuator rod 170 may then be retracted to a position above mating surfaces 162, 163 which in turn allows coupling member 160 to separate from shaft 111 along joining line 165, as illustrated in FIG. 7C.

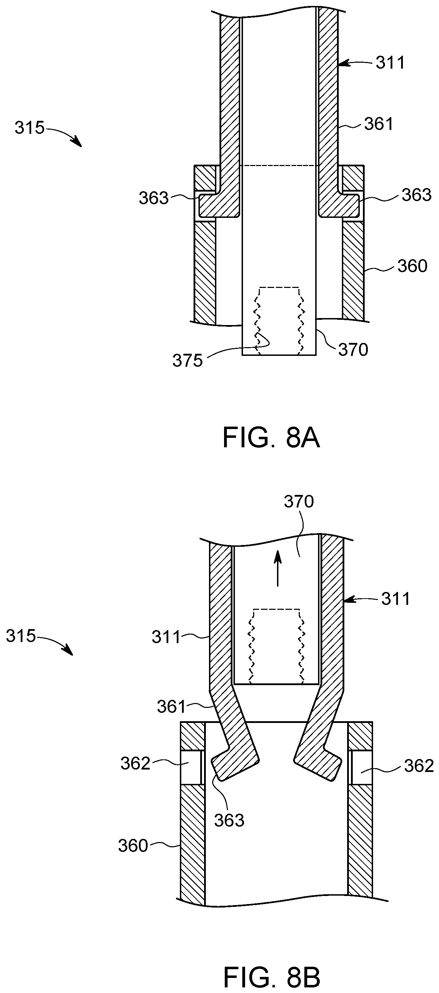

FIGS. 8A and 8B illustrate an alternative example of a coupling system. In this exemplary coupling system 315, shaft 311 of the delivery system (e.g., delivery 600) may be releasably coupled with coupling member 360 via a detent mechanism, for example. In this regard, shaft 311 may form an upper tubular shaft with detent mechanism features and coupling member 360 may form a lower tubular shaft with detent mechanism features configured to releasably connect with the detent mechanism features of shaft 311. In the embodiment depicted, the detent mechanism may include one or more spring arms 361 integrally formed on shaft 311 and one or more receptacles 362 sized to receive spring arms 361 within coupling member 360. However, shaft 311 may include receptacles 362, while coupling member 360 may include spring arms 361, for example. As shown, spring arms 361 may have a flange-like engagement element 363 at a distal end thereof and are preferably biased inwardly, i.e., toward an interior shaft 311, as shown in FIG. 8B. Receptacles or apertures 362 may be configured to receive and mate with respective engagement elements 363 of spring arms 361, as shown in FIG. 8A. Receptacles 362 may extend all the way through the wall of coupling member 360 and may be sized to snuggly fit both engagement elements 362. A snuggly fitting rod (such as actuator rod 370) may extend through shaft 311 and coupling member 360 and may outwardly deflecting the inwardly biased spring arm(s) 361 such that the engagement elements 363 are pushed into respective engagement with a corresponding receptacle 362 thereby coupling the shaft 311 to coupling member 360, as shown in the example of FIG. 8A. When desirable to detach fixation device 112 from delivery 600, actuator rod 370 may be retracted to a position above spring arm(s) 361 and engagement features 363 thereof. This allows the inwardly biased spring arms 361 and corresponding engagement elements 363 to disengage from receptacles 362 thereby detaching shaft 311 and coupling member 360. As mentioned above, actuator rod 370 may be threadedly engaged to stud 131. Thus, actuator rod 370 may first be rotated to unthread its threads 375 from stud 131 and then retracted to release coupling member 360 according to an example of the disclosure.

As mentioned above, fixation device 112 may, in one example, be actuated through multiple positions within a mammalian body during a transcatheter procedure such as by extending and retracting actuator rod 170 when coupled to stud 131 and/or base 139. FIGS. 9A-9B, 10A-10B, 11A-11B, 12A-12B, and FIGS. 13A-13B illustrate several of these possible positions and in a sequence that may be utilized during a transcatheter, therapeutic procedure (e.g., tissue approximation).

FIGS. 9A and 9B depict fixation device 112 in an example of a closed position or delivery position. Fixation device 112 may assume the closed position when being delivered through a guide catheter or sheath 3300 of a steerable guide system. In the closed position, the opposed pair of distal elements 120 may be positioned so that engagement surfaces 125 thereof face each other. The cupped or concave shape of each arm 121 in this example allows arms 121 to surround shaft 111 and optionally contact each other on opposite sides of shaft 111. This provides a low profile for fixation device 112 so that it is readily passable through a delivery catheter 3300 and through any anatomical structures, such as those within the cardiovascular system.

FIGS. 10A-10B depict fixation device 112 in an example of an open position. Fixation device 112 may assume the open position for capturing and grasping leaflets of a heart valve. In an open position, distal elements 120 may be rotated so that engagement surfaces 125 thereof face a first direction such that engagement surfaces 125 are disposed at an acute angle relative to shaft 111. For example, the acute angle formed between each engagement surface 125 and shaft may be 45 degrees to 90 degrees. Stated differently, in the open position, engagement surfaces 125 of distal elements 120 may be oriented 90 degrees to 180 degrees relative to each other. However, it is generally preferable for arms to be positioned 120 degrees relative to each other (and 60 degrees relative to shaft 111) for capturing leaflets. Movement of fixation device 112 from the closed position to the open position may be achieved by advancing stud 131 distally relative to coupling member 160 by distally advancing actuator rod 170. Conversely, fixation device 112 may be moved from the open position to the closed position by retracting actuator rod 170 and retracting stud 131 proximally, according to one example of the disclosure.

As shown in FIG. 10B, proximal elements 140 (or proximal elements 240) may be in a raised or insertion position when fixation device 112 is in the open position to facilitate insertion of leaflets between distal and proximal elements 120, 140 for their capture. A loop 148 may be provided on one or both proximal elements 140 for receipt of a proximal element line that can raise and lower proximal elements 140. Proximal elements 140 are, in one example, biased toward distal elements 120. In this regard, proximal elements 140 may be moved inwardly toward shaft 111 and held against shaft 111 with the aid of proximal element lines 101 which can be in the form of sutures, wires, nitinol wire, rods, cables, polymeric lines, or other suitable structures, as shown in FIG. 10A. Thus, FIGS. 10A and 10B depict fixation device 112 in an insertion configuration in which proximal elements 140 are in a raised position and distal elements 120 are in an open position.

Once fixation device 112 has been positioned in a desired location against the valve leaflets, the leaflets may then be captured between proximal elements 140 and distal elements 120. FIGS. 11A and 11B illustrate fixation device 112 in an example of such a position. Here, proximal elements 140 are lowered toward engagement surfaces 125 so that proximal elements 140 are in a lowered or capture position, and the leaflets are held between distal and proximal elements 120, 140. Proximal elements 140 are, in one example, lowered into the lowered position while distal elements 120 remain in the open position. Thus, fixation device 112, as shown in FIGS. 11A and 11B is in an example of a capture configuration which may be similar to the insertion configuration of FIGS. 10A and 10B, but with the difference being that proximal elements 140 are now lowered toward distal elements 120 by releasing tension on proximal element lines 101 to compress the leaflet tissue therebetween. At any time, the proximal elements 140 may be raised and the distal elements 120 adjusted or inverted to reposition fixation device 112 if regurgitation is not sufficiently reduced according to one example of the disclosure.

FIGS. 12A-12B depict an example of an inverted position of fixation device 112. Fixation device 112 may assume the inverted position to aid in repositioning or removal of fixation device 112. In one example of the inverted position, distal elements 120 may be further rotated from the open position, which may be achieved by advancing stud 131 further relative to the open position, so that the engagement surfaces 125 of distal elements 120 face outwardly, and free ends 121b point distally. Additionally, in some examples, engagement surfaces 125 of each arm 121 may form an obtuse angle relative to shaft 111. For example, the obtuse angle formed between each engagement surface 125 and shaft 111 may be 135 degrees to 180 degrees. Stated differently, in the inverted position, engagement surfaces 125 of distal elements 120 may be oriented 270 degrees to 360 degrees relative to each other.

Also, as shown in FIG. 12B, in one example proximal elements 140 are in their raised position against shaft 111 while distal elements 120 are in the inverted position by exerting tension on the proximal element lines 101. Thus, a relatively large space may be created between proximal and distal elements 140, 18 for repositioning. In addition, the inverted position allows withdrawal of the fixation device 112 through the valve while minimizing trauma to the leaflets. Engagement surfaces 125 provide an atraumatic surface for deflecting tissue as the fixation device is retracted proximally. It should be further noted that tines 145 of proximal elements 140 may, in some examples, be angled slightly in the distal direction (away from the free ends of the proximal elements 140), reducing the risk that tines 145 will catch on or lacerate tissue as fixation device 112 is withdrawn and while proximal elements 140 are in the raised position.

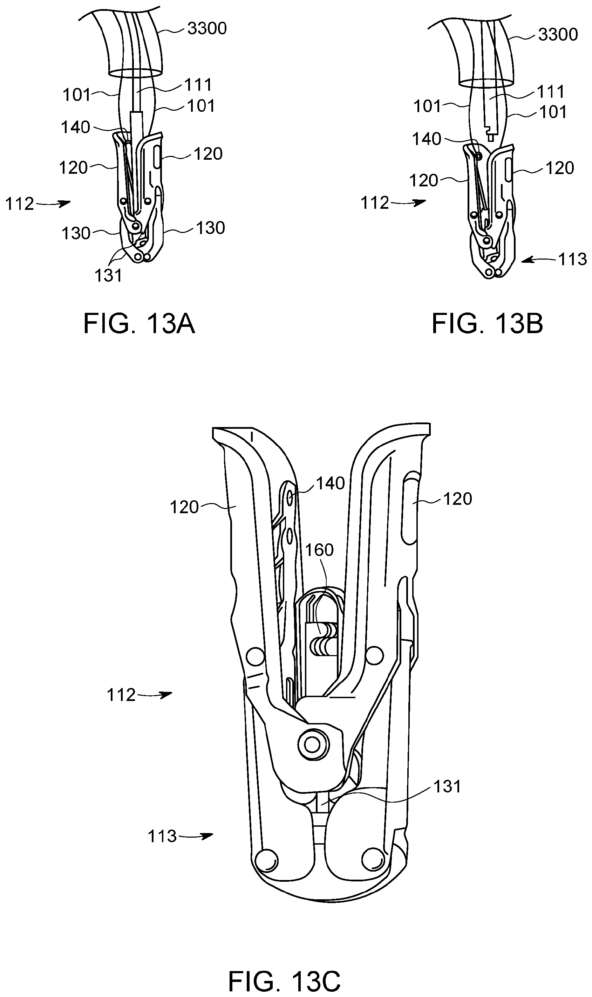

After the leaflets have been captured between distal and proximal elements 120, 140, distal elements 120 may be returned to or toward the closed position where they may be locked in place. An example of such locking is described further below. FIG. 13A illustrates fixation device 112 in the closed position wherein the leaflets (not shown) are captured and coapted. In one example, this is achieved by retraction of the stud 131 proximally relative to coupling member 160 so that the legs 130 of the actuator 113 apply an upwards force to distal elements 120 which in turn rotate distal elements 120 so that engagement surfaces 125 again face one another, similar to that of FIGS. 9A and 9B, and so that distal elements 120 rotate proximal elements 140 in a direction toward shaft 111. However, because the leaflets are captured between distal and proximal elements 120, 140, it may be desirable to keep distal elements 120 at about 20 degrees to 60 degrees relative to each other so as to limit the amount of tension and stress on the native tissue. Thus, while fixation device 112 may be returned to the closed position, such closed position may not be as closed as in the initial delivery position.

As shown in FIG. 13B, fixation device 112 may then be released from shaft 111 of delivery system 600 while in the closed position. As mentioned, fixation device 112 may be releasably coupled to delivery system 600 via a coupling system (e.g., coupling system 115 or 315). When the coupling structures of such coupling system are released, proximal element lines 101 may remain attached to proximal elements 140 following detachment to function as a tether to keep the fixation device 112 connected with the delivery catheter 610 (see FIG. 16) for reconnection and repositioning. However, in other embodiments, proximal elements lines 101 may be released prior to release of fixation device 112 or concurrently with the release of fixation device 112, as described in more detail below.

FIG. 13C illustrates a released fixation device 112 in an example of a closed position. As shown, coupling member 160 remains separated from shaft 111 of delivery system 600, and proximal elements 140 are deployed so that tissue (not shown) may reside between proximal elements 140 and distal elements 120.

As mentioned above, proximal element lines or actuators 101 may be releasably coupled to proximal elements 140. In some examples, proximal element lines 101 may pass through an opening in proximal elements 140, such as openings 146 and 246 in the case of proximal element 240. In other examples, eyelets, which may be formed from one or more lengths of suture, may be coupled to proximal elements 140 and proximal element lines 101 may pass through such eyelets. Thus, proximal element lines 101 may be released from proximal elements 140 prior to, concurrent with, or after release of fixation device 112 from delivery system 600 according to various examples.

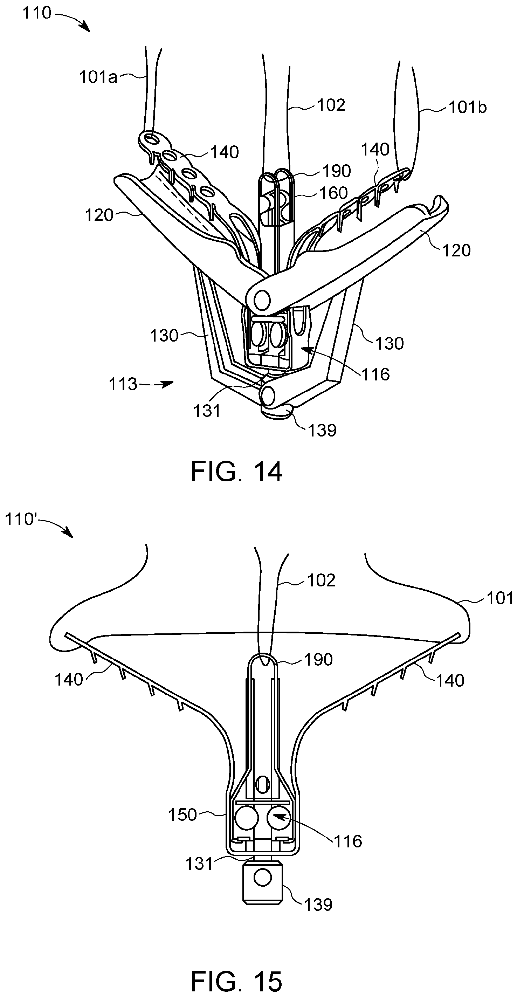

In an exemplary embodiment of interventional tool 110, as shown in FIG. 14, a plurality of proximal element lines 101a, 101b may extend through corresponding lumens 614a, 614b of delivery catheter 610 of delivery system 600 (see FIG. 16) and may be coupled to proximal elements 140 of fixation device 112. Each of proximal element lines 101a and 101b may be elongated flexible threads, wire, cable, sutures, or lines extending through shaft 111, looped through proximal elements 140, and extending back through shaft 111 to a delivery device handle of delivery system 600. When detachment is desired, one end of each proximal element line 101a, 101b may be released from delivery system 600, and the other end pulled to draw the free end distally through shaft 111 and through proximal element 140 thereby releasing it. Also, in this arrangement, proximal element lines 101a and 101b may be independently or concurrently manipulated so as to independently or concurrently raise and lower proximal elements 140, respectively.

In another example, interventional tool 110' may be configured, as shown in FIG. 15 with respect to certain components thereof, such that proximal elements 140 may alternatively be supported by a single proximal element line 101 which may extend through both of the proximal elements 140. In this arrangement both proximal elements 140 may be raised and lowered concurrently by action of a single proximal element line 101. Whether proximal elements 140 are manipulated individually by separate proximal element lines 101 or jointly by a single proximal element line 101, the proximal element lines 101 may extend directly through openings (e.g., openings 146, 246) of the proximal elements 140 and/or through a layer or portion of a covering 117 on proximal elements 140, or through a suture loop/eyelet above or below a covering 117, for example.

In a further example, interventional tool 110" may be configured, as shown in FIG. 16, such that each proximal element line 101a, 101b may be releasably engaged with structures that are activated by removal of the actuator rod 170 that passes through coupling member 160 and shaft 111 such that release of proximal element lines 101a, 101b occurs concurrently with the release of fixation device 112 from delivery system 600. Thus, in one example, which is depicted in FIG. 16, each proximal element line 101a, 101b may have a first end portion 103a (e.g., proximal end portion), which may be coupled to an actuator of a delivery system handle, a second end portion 103b (e.g., distal end portion) which may be releasably engages to shaft 111 via actuator rod 170, and an intermediate portion 103c which may be coupled to a proximal element 140. As described above and as illustrated in FIG. 7A, stud 131 may be releasably attached to actuator rod 170 which passes through coupling member 160 and shaft 111 of delivery system 600. In this way, actuator rod 170 is connectable with fixation device 112 and acts to manipulate fixation device 112 so as to move it through its various positions, which are described above. After the leaflets have been coapted, actuator rod 170 may be removed proximally from stud 131 which may thereby also release coupling member 160 from shaft 111, as described with respect to FIGS. 7A-7C and also FIGS. 8A and 8B with respect to coupling system 315. This action of actuator rod 170 may be utilized to release distal end portion 103b of each of proximal element lines 101a, 101b.

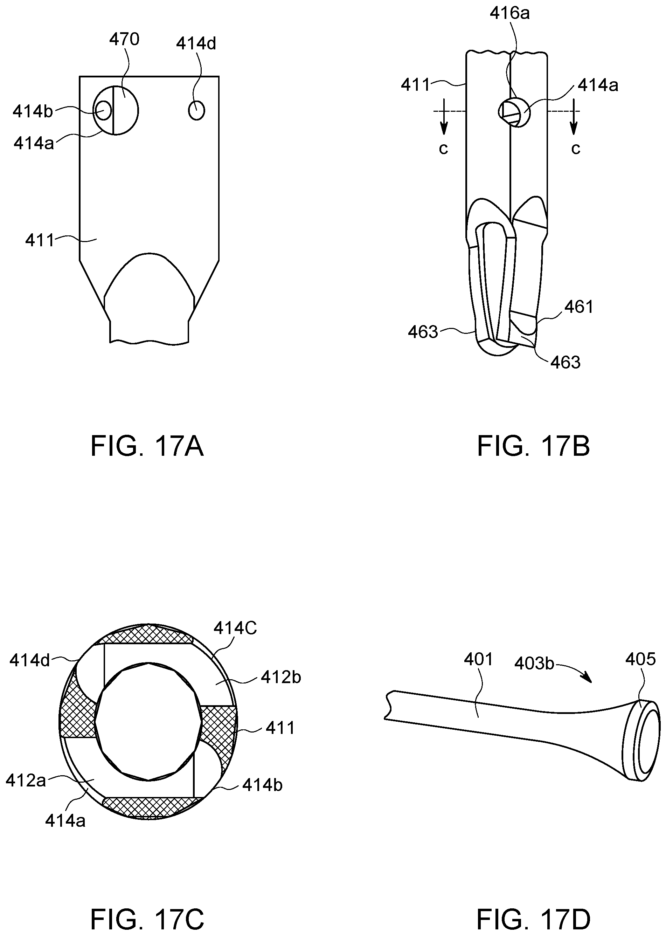

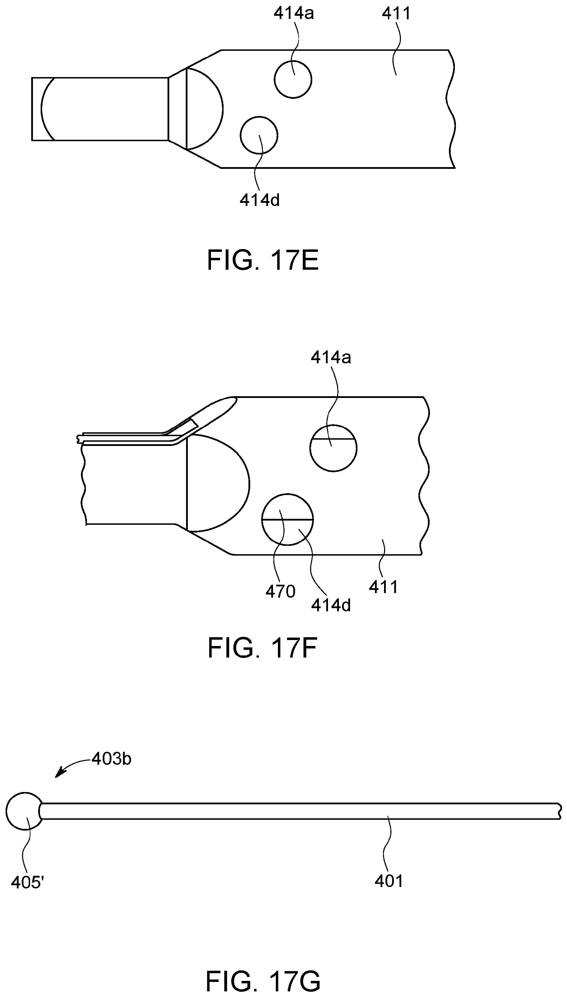

Exemplary features which may be implemented in interventional tool 110" to facilitate release of proximal element lines 101a, 101b in this manner are shown in FIGS. 17A-17G. As depicted, an actuator rod 470 may be used as an anchor to restrict proximal movement of one or more proximal element lines 401. Proximal element line 401 has a distal end portion 403b which may include a catch element 405, for example a trumpet 405 having a cone shape (see FIG. 17D) or other shapes, such as a ball 405' having a spherical shape (see FIG. 17G), which can be sized to be received within shaft 411. As shown in the example of FIGS. 17B, shaft 411 may have spring arms 461 like that of the coupling system 315 of FIGS. 8A and 8B for releasing device 112 from shaft 411. However, shaft 411 may also have mating surfaces of FIGS. 7A and 7C. In any event, a portion of shaft 411 proximal of spring arms 461 (or mating elements 463), may have two slots 412a and 412b defined therein. Slot 412a can define holes 414a and 414b and slot 412b can define holes 414c and 414d. Holes 414a and 414c can be sized to receive catch element 405 of a pair of proximal element lines 401, respectively, therethrough and into slots 412a and 412b, respectively. Holes 414b and 414d can be sized to prevent catch element 405 of proximal element lines 401, respectively, from extending beyond slots 412a and 412b, respectively. The configuration of slots 412a and 412b and holes 414a-414d can allow for easier manufacture of the features in shaft 411. Slots 412a and 412b can be drilled to ensure that slots 412a and 412b do not pass the entire way through shaft 411. In this example configuration, catch elements 405 of proximal element lines 401 can be maintained within shaft 411 to manage the slack of proximal element lines 401.

In one example, catch element 405 of proximal element line 401 can be inserted into slot 412a through hole 414a beyond a longitudinal axis of shaft 411 and toward hole 414b, and catch element 405 of proximal element line 401 can be inserted into slot 412b through hole 414c beyond the longitudinal axis of shaft 411 and toward hole 414d prior to the insertion and coupling of the actuator rod 470 (which passes through shaft 411) with stud 131 of fixation device 112. With actuator rod 470 extending through shaft 411, actuator rod 470 may directly engage catch elements 405 of lines a plurality of proximal element lines 401 thereby preventing their movement back out along the path through which they were inserted. For example, trumpets 405 can be inhibited from being advanced through holes 414b and 414d, respectively, and can be prevented from being pulled past actuator rod 470 and through holes 414a and 414c, respectively. Accordingly, the second end portions 403b of proximal element lines 401 can be held in place relative to shaft 414. Once the actuator rod 470 is decoupled from stud 131 and subsequently retracted, movement of catch elements 405 at the distal end portions of proximal element lines 401 is no longer restricted and proximal element lines 401 are free to move. Upon proximal retraction, proximal element lines 401 can thread through holes 414a and 414c, respectively, and decouple from the proximal elements 140.

In accordance with one example of the disclosed subject matter, slots 412a and 412b can be drilled at an angle towards the distal end of shaft 411 (see FIGS. 17E and 17F), e.g., with hole 414b formed distal to hole 414a on one side, and hole 414d formed distal to hole 141c on the other side. This example configuration of slots 412a and 412b can provide easier deployment of a plurality of proximal element lines 401 and can reduce friction.

Prior to securing second end portion 403b of each proximal element line 401 with the shaft 411, each proximal element line 401 can be coupled with a respective proximal element 140, such as in the manner described above with respect to FIG. 16. Thus, when proximal element lines 401 are actuated proximally, proximal element lines 401 can move proximal elements 140 relative to distal elements 120, thereby moving proximal elements 140 between their respective raised and lowered positions.

As mentioned above, fixation device 112 optionally includes a lock (e.g., lock 116) for locking device 112 in a particular position, such as in any one of the aforementioned open, closed, and inverted positions or any position therebetween. It may be appreciated that according to various examples, lock 116 may be configured for both locking and unlocking which correspondingly allows device 112 to be both locked and unlocked. As described in more detail below with respect to various lock examples, such locks may have components disposed between coupling member 160 and base 139 which may be configured to selectively arrest proximal-distal movement of stud 131/base 139 which consequently arrests movement of distal elements 120. Such locks may help provide end user control of the final arm angle of fixation device 112 for tailored and optimal results for each patient. Additionally, such locks may bring the leaflets and annulus together which may result in beneficial dimensional changes of the target valve which can prevent adverse remodeling of the heart, particularly for patients with heart failure.

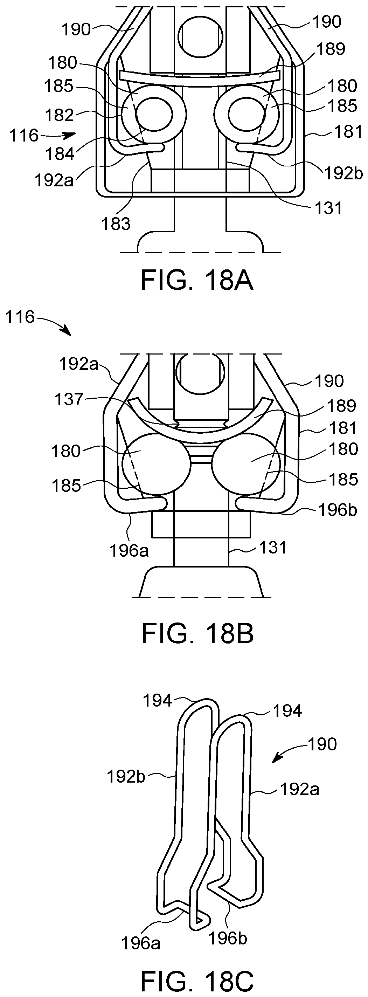

FIGS. 14, 15, and 16A-16C illustrate an embodiment of the lock 116. Lock 116 generally includes a housing 181, one or more wedging elements 180, a release harness 190, and a biasing member 189. Housing 181may be positioned distal to coupling member 160 and may be free-floating, coupled to, or integral with coupling member 160, such as at a distal end thereof. Housing 181 may form a window 183 which may be defined at opposite sides with sloping or tapered surfaces 185 which slope inwardly toward stud 131 in a proximal to distal direction. Wedging elements 180 may be in the form of rolling elements, such as a pair of barbells, disposed on opposite sides of stud 131 and between sloping surfaces 185, as shown in FIGS. 18A and 18B. Each barbell 180 may have a pair of generally cylindrical caps 182 and a shaft 184 therebetween, as illustrated in the barbell cross-section of FIG. 16A. Barbells 180 and stud 131 are preferably comprised of cobalt chromium or stainless steel, however any suitable material may be used. Biasing member 189 may be a spring, such as a leaf spring, for example, and may be positioned at a proximal end of housing 181 between sloping surfaces 185 and proximal to barbells 180 such that spring 189 bears on barbells 180 and biases them in a distal direction. Thus, when barbells 180 are pushed distally by spring 189, they are correspondingly pushed inwardly and wedged against stud 131 by sloping surfaces 185, as illustrated by FIG. 18A, which depicts barbells 180 in a proximal and unlocked position, and FIG. 18B, which depicts barbells 180 in a distal and locked position.

As shown in FIGS. 14, 15, and 18C, release harness 190 may be in the form of a ridged wire or rod that may extend proximally from stud 131 toward a proximal end of fixation device 112 and at opposite sides thereof. In this regard, release harness 190 may form a first portion or front portion 192a and a second portion or rear portion 192b. Each of first and second portions 192a, 192b may include a crest or closed proximal end 194 through which a lock line 102 may be threaded and engaged, as described below. Release harness 190 may also form hooked distal ends 196a, 196b which may extend between first and second portions 192a, 192b and between sloping surfaces 185 and stud 131, as shown in FIGS. 18A and 18B. Thus, hooked ends 196a and 196b may be moveable proximally-distally within window 183 formed between sloped surfaces 185 and stud 131. Additionally, hooked ends 196a and 196b may be positioned distal of barbells 180 such that pulling up on harness 190 moves hooked ends 196a, 196b proximally so as to push the respective barbells 180 against the bias of spring 189 and move them to their unlocked position.

Movement of harness 190 may be performed by one or more lock line 102 which may be coupled to harness 190 by such as by threading lock line 102 through and engaging one or more of proximal ends 194 of first and second portions 192a, 192b thereof, as shown in FIGS. 14 and 15. Such lock line 102 may have a first end 102a fixedly secured to a delivery system handle 1012 of delivery system 600 and a second end 102b releasably secured to a delivery system handle 1012, as described in more detail below. In this regard, tension can be selectively applied to lock line 102 to unlock and lock the lock 116. Also, lock line 102 can be released from release harness 190 prior to, concurrently with, or after release of fixation device 112 from delivery system 600 which may be achieved by releasing the second end 102b from delivery system handle and pulling lock line 102 and its second end through shaft 111. Lock line 102 may be comprised of any suitable material, typically wire, nitinol wire, cable, suture, or thread, to name a few. In addition, lock line 102 may include a coating, such as parylene. Parylene is a vapor deposited pinhole free protective film which is conformal and biocompatible. It is inert and protects against moisture, chemicals, and electrical charge.

When an upwards force is applied to harness 190 by the lock line 102, hooked ends 196a, 196b may raise barbells 180 against spring 189, as shown in FIG. 18A. This may draw barbells 180 up along sloping surface 185 which unwedges barbells 180 from against stud 131. In this position, stud 131 is free to move. Thus, when lock line 102 is tensioned to raise or lift harness 190, lock 116 is in an unlocked position wherein stud 131 is free to move actuator 113 and therefore distal elements 120 to any desired position. Releasing tension in lock line 102 may, on the other hand, transition the lock 116 to a locked position, as shown in FIG. 18B. Thus, by releasing the upwards force on barbells 180 by hooked ends 192a, 192b, spring 189 forces barbells 180 downwards and wedges barbells 180 between a sloping surface 185 and stud 131. This restricts motion of stud 131, which in turn locks actuator 113 and therefore distal elements 120 in place. In addition, stud 131 may include one or more grooves or indentations 137 which may receive shaft 184 of each barbell 180. This may provide more rapid and positive locking by causing barbells 180 to settle in a definite position, increase the stability of lock 116 by further preventing movement of barbells 180, as well as tangible indication to the user that each barbell 180 has reached a locking position. In addition, grooves 137 may be used to indicate the relative position of distal elements 120, particularly the distance between distal elements 120. For example, each groove 137 may be positioned to correspond with a 0.5- or 1.0-mm decrease in distance between distal elements 120. As stud 131 is moved, barbells 180 may contact grooves 137, and by counting the number of grooves 137 that are felt as stud 131 is moved, the user can determine the distance between distal elements 120 and can provide the desired degree of coaptation based upon leaflet thickness, geometry, spacing, blood flow dynamics and other factors. Thus, grooves 137 may provide tactile feedback to the user.

Lock 116 allows fixation device 112 to remain in an unlocked position when attached to delivery system 600 during grasping and repositioning and then maintain a locked position when left behind as an implant. It may be appreciated, however, that lock 116 may be repeatedly locked and unlocked throughout the placement of the fixation device 112 if desired. Once the final placement is determined, lock line 102 may be removed and fixation device 112 may be left behind.

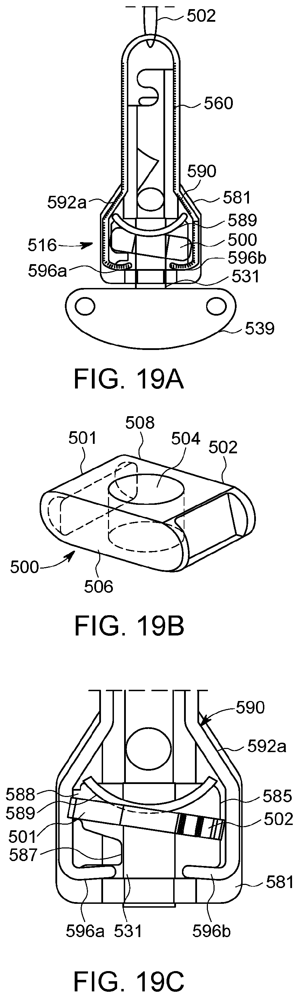

FIGS. 19A-19C depict a lock 516 according to another embodiment of the present disclosure that may be incorporated into a fixation device 112 of the disclosure. In this embodiment, lock 516 also includes a housing 581, a spring 589, a release harness 590, and a wedging element 500. However, instead of sloping surfaces 185 as present in the example of locking element 116, housing 185 may include generally parallel sidewalls 585 and may include a finger or protrusion 587 extending from one of sidewalls 585 toward stud 131, as best shown in FIG. 19C. Such finger 587 may slope in a distal direction and may define a proximal notch 588. Also, as shown in FIG. 19C, first hooked end 596a of release harness 590 may be positioned distal of finger 587.

Furthermore, wedging element may comprise a binding lever or binding plate 500. As shown in FIG. 19B, binding plate 500 may have an oblong shape that may extend lengthwise between a first end 501 and a second end 502 thereof. An aperture 504 may be formed between first and second ends 501, 502 and may extend from a top planar surface 508 through a bottom planar surface 506 of binding plate 500. Binding plate 500 may be positioned between sidewalls 406 so that stud 131 passes through aperture 504 and so that first end 501 of binding plate 500 is positioned within notch 588 proximal of finger 587, as best shown in FIG. 19C. Thus, finger 587 may be positioned between first end 501 of binding plate 500 and first hooked end 596a of released harness 590. Also, spring 589 may be positioned proximal to binding plate 500 and provide downward or distal bias thereto. Binding plate 500 and stud 131 may be comprised of any suitable material. In some embodiments, binding plate 500 may have a higher hardness than stud 131. In other embodiments, binding plate 500 may be comprised of a flexible or semi-flexible material. Such flexibility may allow slight movement of stud 131 in the proximal and distal directions, therefore allowing slight movement of distal elements 120 when lock 516 is in the locked position. This may allow fixation device 112 to adjust in response to dynamic cardiac forces.

FIGS. 19A and 19C illustrate binding plate 500 in a locked position or configuration. In this regard, spring 589 pushes binding plate 500 in a distal direction. However, because first end 501 of binding plate 501 is positioned within notch 588, axial movement of first end 501 toward a distal end of housing 581 is prohibited while axial movement of second end 502 of binding plate 500 is permitted. Thus, finger 587 obstructs first end 501 from axial movement and creates a lever type movement of binding plate 500. Moreover, finger 587 obstructs first hooked end 596a of release harness 590 from axial movement resulting in a side-to-side pivoting of release harness 590 upon tension of lock line 102. This pivoting movement correspondingly results in second hooked end 596b of release harness moving proximally and controlling movement of second end 502 of binding plate 500. As such, when an upwards force is applied to harness 590 by lock line 102, second hooked end 596b of release harness 590 raises second end 502 of plate 500 against spring 589 so that planar surfaces 506, 508 of binding plate 500 become oriented substantially perpendicular to stud 131. This aligns aperture 504 with stud 131 allowing free movement of stud 131 in the proximal-distal direction. Thus, in this state, lock 516 is unlocked wherein stud 131 is free to move actuator 113 and therefore distal elements 120 to any desired position.

Release of harness 590 by lock line 102 transitions lock 516 back to the locked position. By releasing the upwards force on second end 502 of binding plate 500, spring 589 forces second end 502 of biding plate 500 downwards, which misaligns aperture 504 relative to stud 531, and correspondingly wedges binding plate 500 against stud 131, as best shown in FIG. 19C. This arrests movement of stud 131, which in turn locks actuator 113 and therefore distal elements 120 in place. It may be appreciated that binding plate 500 may have any suitable form to function as described above. For example, plate 500 may have a variety of shapes with or without planar surfaces 506, 508 and/or the aperture 504 may be of a variety of shapes and positioned in a variety of locations, to name a few. For example, binding plate 500 may not have a through-hole, like that of aperture 504, but may rather have a notch such that binding plate 500 does not encircle stud 131 but rather partially surrounds it. Further, it may be appreciated that any number of binding plates 500 may be present. Each binding plate 500, in this regard, may provide an additional binding location which may enhance lock performance.

While the above-described nonlimiting examples of fixation device 112 may utilize a push-to-open, pull-to-close mechanism for opening and closing distal elements 120, it should be understood that a pull-to-open, push-to-close mechanism may alternatively be provided. For example, distal elements 120 may be coupled at their proximal ends to stud 131 rather than to coupling member 160, and legs 130 may be coupled at their proximal ends to coupling member 160 rather than to stud 131. In this example, when stud 131 is pushed distally relative to coupling member 160, distal elements 120 may close, while pulling on stud 131 proximally toward coupling member 160 may open distal elements 120. Regardless, the aforementioned lock examples may be configured to arrest stud to lock distal elements 120 in the desired position, as described.

It is to be understood that the fixation devices and components thereof described above are provided as examples are not to be considered as limiting to fixation devices suitable for use with other aspects of the disclosure.

During a TEER procedure, a fixation device, such as fixation device 112, may unintentionally snag or engage anatomic tissue structures, such as chordae tendineae CT, papillary muscles PM, or leaflets LF, for example. This can occur, for example, when distal elements 120 are in an open position and proximal elements 140 are in the raised position which may expose frictional elements 145 to tissue structures which may then become inadvertently caught on frictional elements 145.

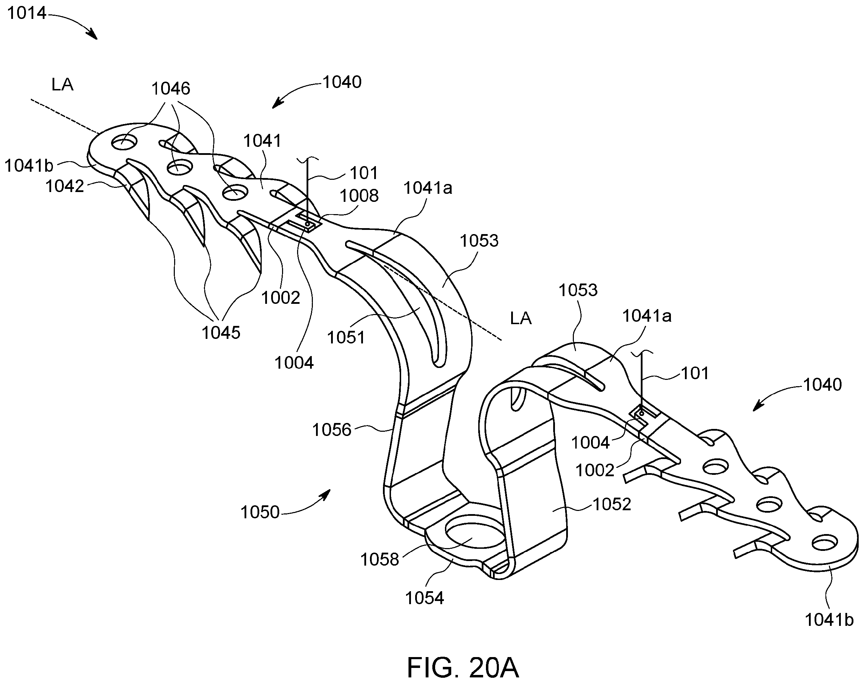

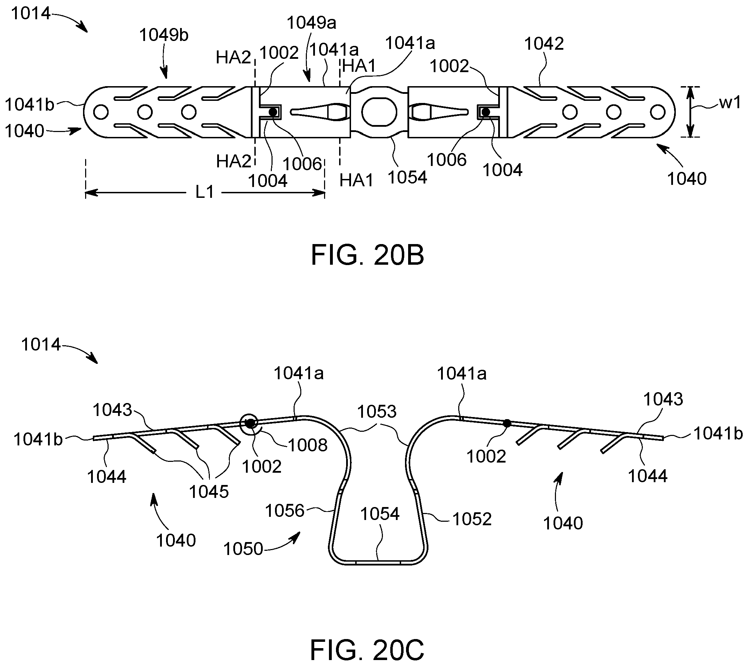

Referring now in addition to FIGS. 20A-20E, which depict a gripping device 1014 according to another embodiment of the present disclosure, which may be incorporated into any of the fixation devices of the disclosure. Gripping device 1014 may be configured to fold over itself so as to shield frictional elements 1045 from tissue structures, such as the chordae CT, papillary muscles PM, and leaflets LF, for example.

Gripping device 1014 is similar to gripping device 114 except for differences explicitly described and/or shown and may include any features or characteristics of any other gripping device of the disclosure. Thus, like elements are accorded like reference numerals to that of gripping device 114 but within the 1000-series of numbers, meaning base section 1050 is alike to base section 150, arm bend features 1053 are alike to arm bend features 153, and so on, except for differences explicitly described and/or shown. In addition to base section 1050 and arm bend features 1053, gripping device 1014 generally includes a pair of proximal elements 1040.

As shown, each proximal element 1040 may include an elongate arm 1041 that has a first length L1 extending from a first end portion or fixed end 1041a and a second end portion or free end 1041b and a first width W1 extending transverse to longitudinal axis LA. Width W1 may be dimensioned such that proximal element 1040 is recessed within a tissue a corresponding distal element, such as distal element 120, when in a lowered position. First end portion 1041a may be connected to and extend from a corresponding arm bend feature 1053 which may be connected to and extend from a base section 1050. Arm bend features 1053 and base section 1050 are similar to arm bend features 153 and base section 150 of gripping device 114, which is described above. In this regard, arm bend features 153 may each be configured to bend a corresponding proximal element 1040 about a first hinge axis HA1 between a raised position and a lowered position with respect to a corresponding distal element, such as distal element 120, for example. Exemplary raised and lowered positions are described above with particular reference to FIGS. 10A-11B. Additionally, proximal element 1040 may include a plurality of frictional elements 1045 extending from a distal surface 1044 thereof. For example, elongate arm 1041 may include opposed side edges 1042 each forming a plurality of frictional elements 1045 (only one of which is referenced for ease of illustration), such as by cutting and bending the base material forming proximal elements 1040, for example. In other embodiments, frictional elements 1045 may be connected to or extend from distal surface 1044 of elongate arm 1041 at locations between first and second side edges 1042 and between first and second end portions 1041a, 1041b of elongate arm 1041.

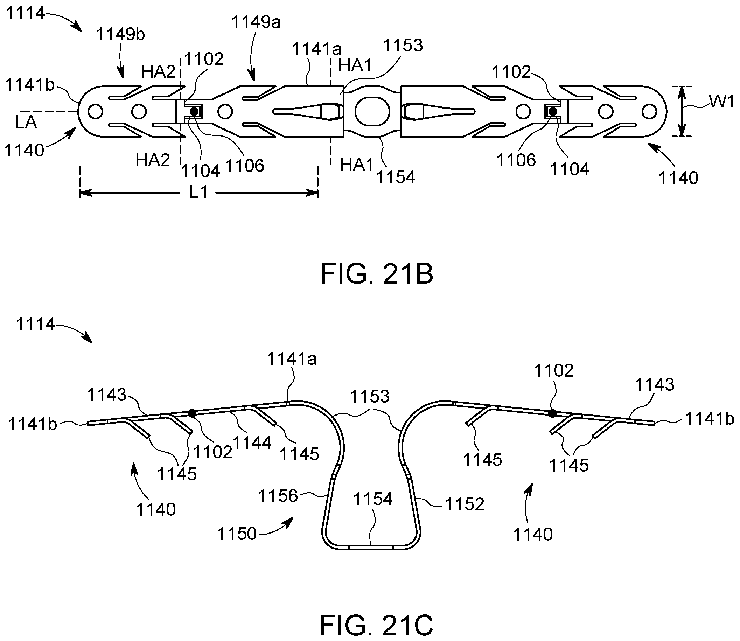

As mentioned above, each proximal element 1040 may be configured to fold over itself to shield the distal side of elongate arm 1041 so that tissue does not become snagged on frictional elements 1045. In this regard, proximal elements 1040 may each include a hinge 1002 positioned between first end portion 1041a and second end portion 1041b. Hinge 1002 may be an integral hinge, such as a living hinge, or may be a modular hinge, such as a hinge pin, for example. Hinge 1002 may extend transverse to a longitudinal axis LA of proximal element 1040 and from one side edge 1042 to another side edge 1042 of elongate arm 1041. As such, hinge 1002 may divide proximal element 1040 into a first section 1049a and a second section 1049b. First section 1049a may extend between hinge 1002 and fixed end 1041a, and second section 1049b may extend between hinge 1002 and free end 1041b. For example, hinge 1002 may be closer to first end portion 1041a than second end portion 1041b, such as between 25% and 50% of the first length L1. In the embodiment depicted, all of frictional elements 1045 of each proximal element 1040 may be disposed within second section 1049b while first section 1049a may not include any frictional elements 1045. In this regard, second section 1049b may include a plurality of frictional elements 1045, such as two to twelve frictional elements 1045, for example. Frictional elements 1045 may be arranged in rows such that each row includes a plurality of frictional elements 1045, such as two to four frictional elements 1045 per row, for example. In the embodiment depicted, second section 1049b may include three rows of two frictional elements 1045 each for a total of six frictional elements 1045. Additionally, hinge 1002 may be biased so as to bias second section 1049b in alignment with first section 1049a as shown in FIG. 21C. Such bias may be an inherent material property within hinge 1002, such as when hinge 1002 is a living hinge. For example, hinge 1002 may be made from a memory metal material, such as nitinol, for example, which may be constructed to have a memory that returns second section 1049b into alignment with first section 1049a. Alternatively, hinge 1002 may have a biasing element 1008, such as a torsion spring, for example, that biases second section 1049b into alignment with first section 1049a.

Proximal element 1040 may further include a lever or beam 1004 (also referred to herein as a pull tab) which may include one or more openings 1006 therein to receive a first proximal element line, such as line 101, as shown in FIG. 20A. Proximal element 1040 may also include one or more other openings 1046, such as within second section 1049b which may receive a second proximal element line (e.g., line 101) such that the second proximal element line may raise and lower proximal element 1040 while the first proximal element line 101 connected to lever 1004 may be used to rotate second section 1049b relative to first section 1049a. As shown, lever 1004 may be connected to hinge 1002 and extend therefrom along longitudinal axis LA. In the embodiment depicted, lever 1004 may extend in a direction toward first end portion 1041b such that pulling lever 1004 proximally rotates second section 1049b distally about hinge 1002 and about a second hinge axis HA2. Second hinge axis HA2 may be offset from first hinge axis HA1 in the longitudinal direction and may be parallel to first hinge axis HA1. However, in other embodiments, second hinge axis HA2 may be angled relative to first hinge axis HA1. Lever 1004 may be recessed within a recess 1008 of first section 1049a such that lever 1004 is flush with or recessed within a proximal surface 1043 of first section 1049a when proximal element 1040 is in a first configuration, as described in more detail below. Such recess 1008 may extend entirely through proximal element 1040 from proximal surface 1043 to distal surface 1044. However, in other embodiments recess 1008 may extend through proximal surface 1043 but stop short of extending through distal surface 1044 which may provide an abutment for lever 1004 to help prevent second section 1049b from rotating proximally when engaging tissue. Hinge 1002 and lever 1004 may be tuned to tailor the amount of line tension needed to overcome the bias of hinge 1002.

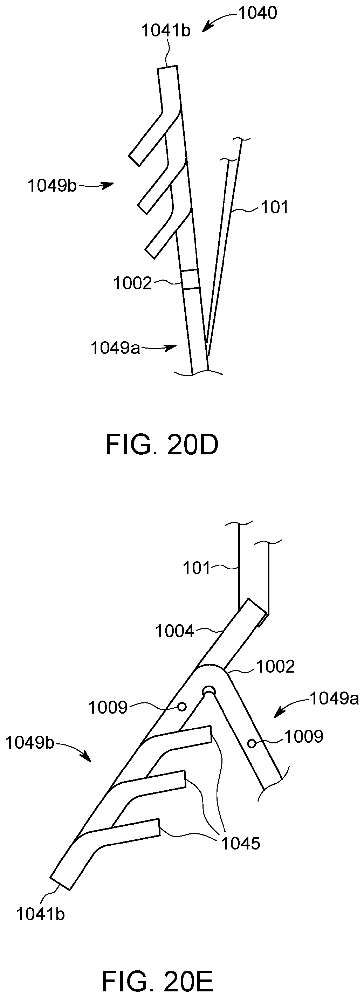

Each proximal element 1040 may have a first state, a second state, and a third state. In the first state or un-tensioned state, as shown in FIG. 20C, proximal element 1040 is in a lowered position in accordance with a bias of bend feature 1053 to capture tissue. When in the first state, first and second sections 1049a, 1049b align such that they are coaxial with longitudinal axis LA. In the second state or tensioned state, as illustrated in FIG. 20D, proximal element line 101, which may be connected to lever 1004 or one of openings 1046, is tensioned so that proximal element 1040 overcomes the bias of bend feature 1043 and is in the raised position. However, in the second state, first and second sections 1049a, 1049b remain aligned as in the first state. Thus, proximal element 1040 has a first configuration in which first and second sections 1049a, 1049b are aligned, and proximal element 1040 may be in the first configuration when it is in the first state and the second state. The tension of proximal element line 101 for transitioning proximal element 1040 from the first state to the second state may be lower than the tension used to transition proximal element 1040 to the third state.

Proximal element 1040 may be transitioned to the third state or high-tension state when it may be desirable to shield frictional elements 1045 from tissue, such as when maneuvering a fixation device, such as fixation device 112, relative to valve leaflets while proximal element 1040 is in the raised position and distal elements 120 are in an open position. To transition proximal element 1040 to the third state, tension in proximal element line 101 is increased relative to that of the second state to overcome the bias of hinge 1002 and rotate second section 1049b downwardly or distally about second hinge axis HA2. Thus, proximal element has a second configuration which it assumes in the third state (i.e., high-tension state). In the second configuration, as shown in FIG. 20E, second section 1049b is angled relative to first section 1049a such that frictional elements 1045 face or point toward first section 1049a and lever 1004 is moved to a location outside of recess 1008. In this regard, transitioning proximal element 1040 to the second configuration (i.e., third state) presents proximal surface 1043 of proximal element 1040 to surrounding tissue and shields frictional elements 1045 from such tissue to help mitigate or prevent inadvertent tissue engagement. When the risk of inadvertent tissue engagement is low (e.g., when the fixation device is at the desired position within the valve) and it is time to capture tissue, the excess tension in proximal element line 101 may be released allowing the bias of hinge 1002 to return proximal element 1040 to the first configuration (i.e., first state or second state) and second section 1049b into alignment with first section 1049a.

Additionally, in some embodiments, first and/or second sections 1049a, 1049b may include radiopaque indicators or markers 1009, as shown in FIG. 20E. Such indicators 1009 may be made from a radiopaque material, such as platinum, platinum-iridium, or tantalum for example. Indicators 1009 may make it easier to verify the transition between first and second configurations via medical imaging, such as fluoroscopy.

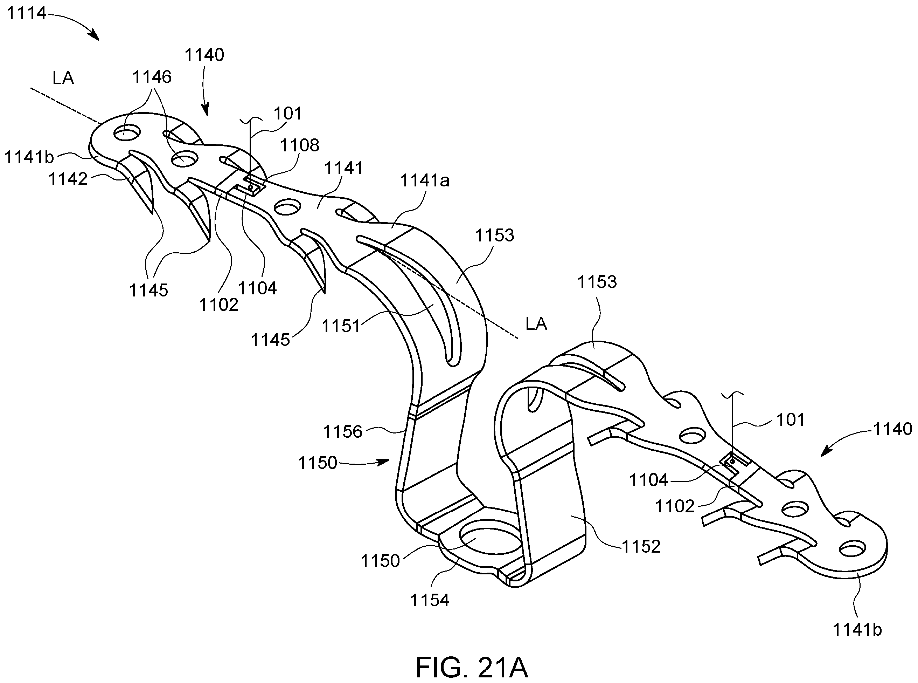

Referring now in addition to FIGS. 21A-21C, which depict one example of a gripping device 1114 according to another embodiment of the present disclosure, which may be incorporated into any of the fixation devices of the disclosure. Gripping device 1114 is similar to gripping device 1014 except for differences explicitly described and/or shown and may include any features or characteristics of any other gripping device of the disclosure. Thus, like elements are accorded like reference numerals to that of gripping device 1014 but within the 1100-series of numbers. For instance, gripping device 1114 is like gripping device 1014 in that it may include a base section 1150, proximal elements 1140, and arm bend features 1153 defining a first hinge axis HA1 about which each proximal element 1140 rotates between a first configuration and a second configuration. Additionally, each proximal element 1140 may include a hinge 1102, which may be a living hinge or a modular hinge, and a lever 1104 attached to hinge 1102. Hinge 1102 may define a second hinge axis HA2 and may divide proximal element 1140 into a first section 1149a and a second section 1149b such that second section 1149b may fold or bend relative to first section 1149a to shield frictional elements 1145 of proximal element from inadvertently engaging tissue.

However, unlike gripping device 1014, first and second sections 1149a, 1149b of each proximal element 1140 may include a plurality of frictional elements 1145 extending distally therefrom. In this regard, hinge 1102 of each proximal element 1140 may be closer to second end portion 1141b than proximal element 1040, such that hinge 1102 may be located at or near 50% of the first length L1 of proximal element. In other embodiments, hinge may be located between 50% and 75% of first length L1, for example. Thus, when proximal element 1140 is actuated via a proximal element line 101 to its second configuration, second section 1149b may rotate about second hinge axis HA2 downwardly so that second section 1149b overlaps first section 1149a and frictional elements 1145 of first and second sections 1149a, 1149b face each other thereby shielding them from surrounding tissue.

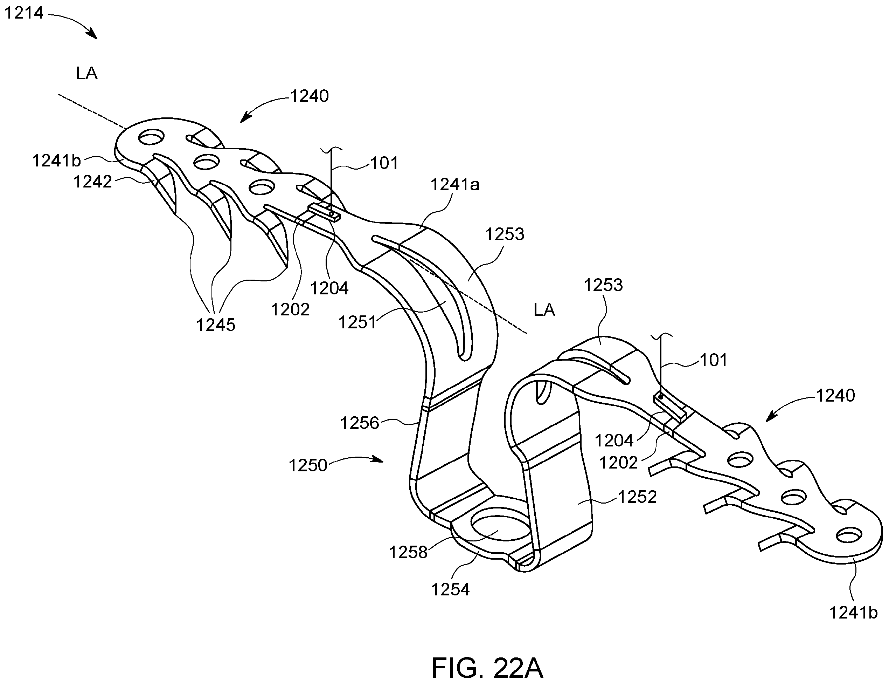

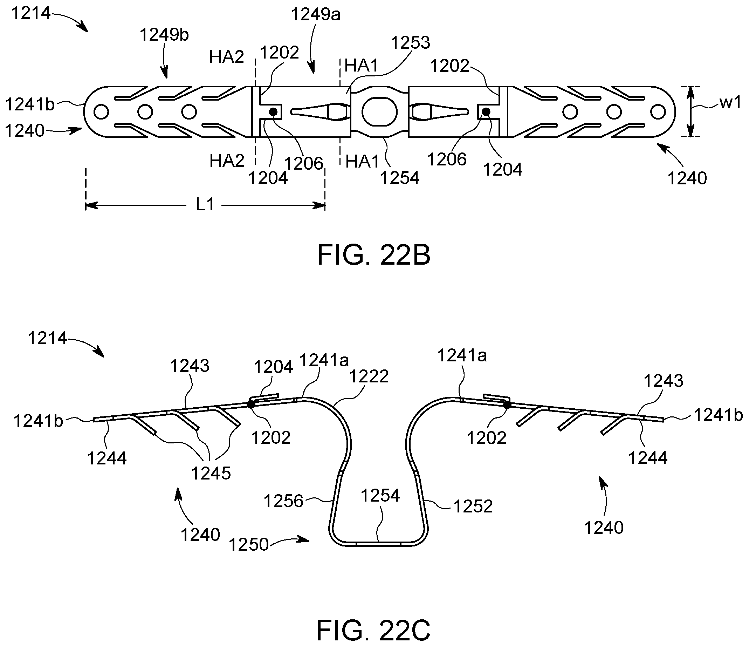

Referring now in addition to FIGS. 22A-22D, which depict one example of a gripping device 1214 according to another embodiment of the present disclosure, which may be incorporated into any of the fixation devices of the disclosure. Gripping device 1214 is similar to gripping device 1014 except for differences explicitly described and/or shown and may include any features or characteristics of any other gripping device of the disclosure. Thus, like elements are accorded like reference numerals to that of gripping device 1014 but within the 1200-series of numbers. For instance, gripping device 1214 is like gripping device 1014 in that it may include a base section 1250, proximal elements 1240, and arm bend features 1253 defining a first hinge axis HA1 about which each proximal element 1240 rotates between a raised position and a lowered position. Additionally, each proximal element 1240 may include a hinge 1202, which may be a living hinge or a modular hinge, and a lever 1204 attached to hinge 1202. Hinge 1202 may define a second hinge axis HA2 and may divide proximal element 1240 into a first section 1249a and a second section 1249b such that second section 1249b may fold or bend relative to first section 1249a to shield frictional elements 1245 of proximal element from inadvertently engaging tissue.

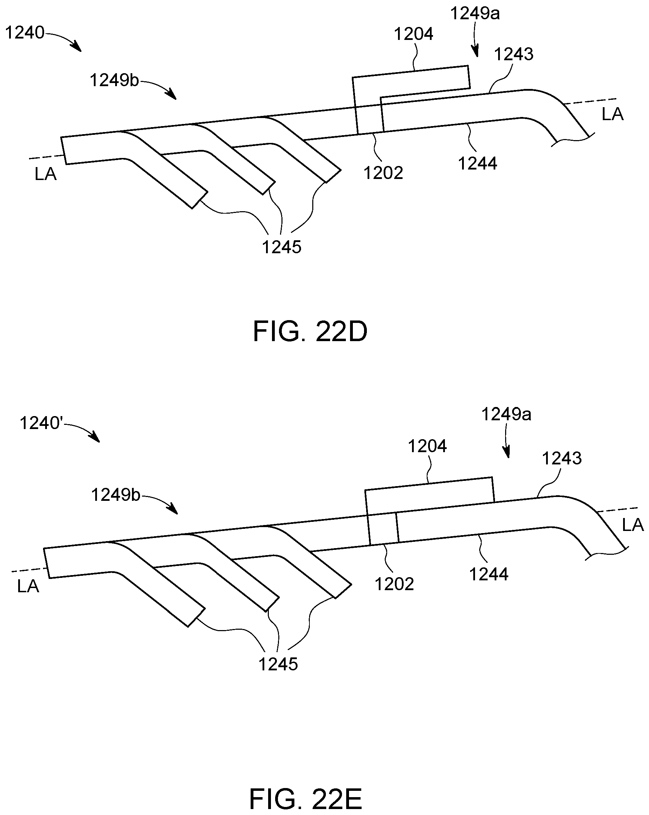

However, unlike gripping device 1014, lever 1204 of each proximal element 1240 may not be recessed within a recess within first section 1249a. Instead, lever 1204 may sit proud of proximal surface 1243 of proximal element 1240. In other words, when first and second sections 1249a, 1249b are in the first configuration, lever 1204 may extend above or proximal to proximal surface 1243 of first section 1249a, as best shown in FIG. 22D. In this regard, when proximal element 1240 engages tissue, pressure from contact with the tissue may have a tendency to rotate second section 1249b upwardly about second hinge axis HA2. This may correspondingly rotate lever 1204 downwardly toward first section 1249a such that lever 1204 may contact or abut proximal surface 1243 of first section 1249a thereby providing resistance against further proximal rotation of second section 1249b.

As shown in FIG. 22D, lever 1204 may be slightly offset from proximal surface 1243 of first section 1249a which may allow for minimal proximal rotation of second section 1249b about second hinge axis HA2. However, in some embodiments, such as in proximal element 1240' shown in FIG. 22E, lever 1204 may sit flush against proximal surface 1243 of first section 1249a when first and second sections 1249a, 1249b are aligned so as to prohibit any proximal rotation of second section 1249b. Although lever 1204 is shown as having a width less than first width W1 of proximal element, in some embodiments, lever 1204 may span a majority or the entirety of width W1 of proximal element 1240 so as to distribute resistance forces across width W1.

Gripping devices 1014, 1114, and 1214 may each be separately utilized in conjunction with distal elements 120 of fixation device 112, as an example. Additionally, while proximal elements 1040, 1140, and 1240 are depicted as being connected to and extending from corresponding arm bend feature 1053, 1153, and 1253 and base sections 1050, 1150, and 1250, which are similar to arm bend feature 153 and base section 150 of FIGS. 5A and 5C, it should be understood that proximal elements 1040, 1140, and 1240 can be connected to and extend from arm bend feature 260 and second arm 250 of FIGS. 6A and 6B or bend feature 260 and second arm 250' of FIG. 6C, for example. Thus, proximal elements 1040, 1140, and 1240 may be adapted to couple to a center portion of a fixation device, like in fixation device 112, or adapted to couple directly to a distal element of a fixation device as has been described with respect to gripping devices 214 and 214' of FIGS. 5A-5B and 6A-6C.

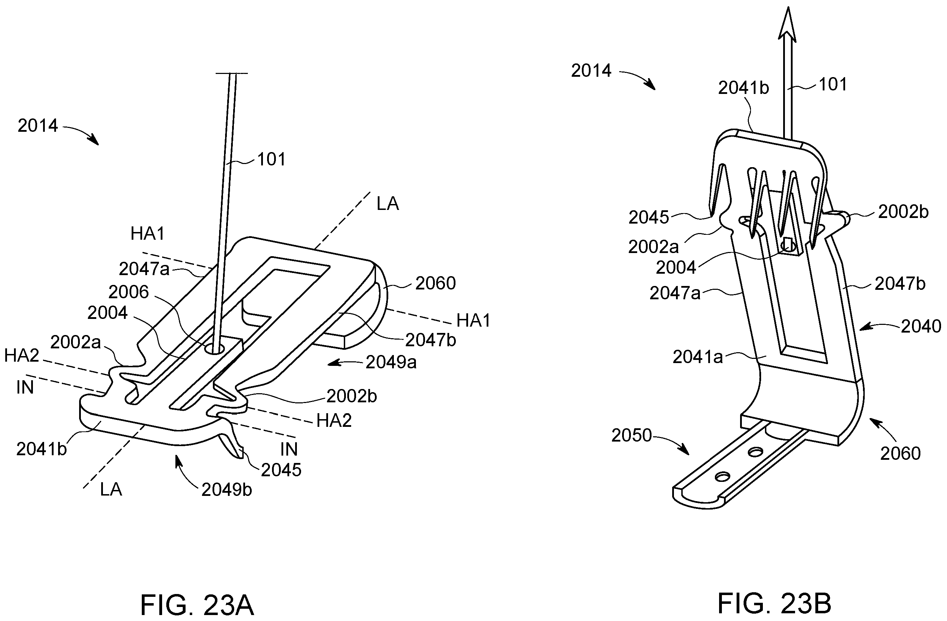

Referring now in addition to FIGS. 23A-23C, which depict one example of a gripping device 2014 according to another embodiment of the present disclosure, which may be incorporated into any of the fixation devices of the disclosure. Gripping device 2114 is similar to gripping device 214 except for differences explicitly described and/or shown and may include any features or characteristics of any other gripping device of the disclosure. Thus, like elements are accorded like reference numerals to that of gripping device 214 but within the 2000-series of numbers, meaning base section 2050 is alike to base section 250, arm bend feature 2060 is alike to arm bend feature 260, and so on, except for differences explicitly described and/or shown. In addition to base section 2050 and arm bend feature 2060, gripping device 2014 includes a proximal element 2040.

As shown, proximal element 2040 may extend from a first end portion 2041a to a second end portion 2041b. First end portion 2041a may be a fixed end coupled to arm bend feature 2060 and second end portion 2041b may be a free end. Arm bend feature 2060 may be configured to bend proximal element 2040 about a first hinge axis HA1 between a raised position and a lowered position with respect to a corresponding distal element, such as distal element 120, for example. Proximal element 2040 may include a pair of elongate member or struts 2047a, 2047b each coupled to and extending between first and second end portions 2041a, 2041b. Elongate members 2047a, 2047b may be offset from each other in a direction transverse to a longitudinal axis LA so as to define a space 2048 therebetween. Additionally, a plurality of frictional elements 2045 may extend distally from proximal element 2040. In the embodiment depicted, frictional elements 2045 may extend from second end portion 2041b and may be arranged in a single row of four frictional elements 2045. However, in other embodiments more or less frictional elements 2045 may be included in the row of frictional elements 2045 and two or more rows may also be provided. In further embodiments, one or more frictional elements 2045 may extend from each elongate member 2047a, 2047b.

Proximal element 2040 may be configured to fold over itself to shield the distal side of proximal element 2040 so that tissue does not become snagged on frictional elements 2045. In this regard, proximal element 2040 may include a first hinge 2002a and a second hinge 2002b positioned on respective elongate members 2047a, 2047b. Hinges 2002a, 2002b may each be an integral hinge, such as a living hinge, or may each be a modular hinge, such as a hinge pin, for example. In the embodiment depicted, each hinge 2002a, 2002b is a living hinge which may have an undulating profile such that each hinge projects outwardly and/or inwardly relative to elongate members 2047a, 2047b. Such undulating profile may help facilitate bending at second hinge axis HA2. Hinges 2002a, 2002b may be aligned with each other in the transverse direction. As such, hinges 2002a, 2002b may together define a second hinge axis HA2 and may divide proximal element 2040 into a first section 2049a and a second section 2049b. First section 2049a may extend between hinges 2002a, 2002b and first end portion 2041a, and second section 2049b may extend between hinges 2002a, 2002b and second end portion 2041b. For example, hinges 2002a, 2002b may be closer to second end portion 2041b than first end portion 2041a, as shown. In another example, hinges 2002a, 2002b may be located midway between first end portion 2041a and second end portion 2041b. In a further example, hinges 2002a, 2002b may be closer to first end portion 2041a than second end portion 2041b. In the embodiment depicted, all of frictional elements 2045 may be disposed within second section 2049b while first section 2049a may not include any frictional elements 2045. Additionally, hinges 2002a, 2002b may be biased so as to bias second section 2049b in alignment with first section 2049a as shown in FIGS. 23A and 23B. Such bias may be an inherent material property within each hinge 2002a, 2002b, such as when hinges 2002a, 2002b are a living hinge. For example, hinges 2002a, 2002b may be made from a memory metal material, such as nitinol, for example, which may be constructed to have a memory that returns second section 2049b into alignment with first section 2049a. Alternatively, each hinge 2002a, 2002b may have a biasing element, such as a torsion spring, for example, that biases second section 2049b into alignment with first section 2049a.

Proximal element 2040 may further include a lever or beam 2004 which may include one or more openings 2006 therein to receive a first proximal element line, such as line 101, as shown in FIG. 23A. As shown, lever 2004 may connect to second end portion 2041b of proximal element 2040 and define an interface IN between lever 2004 and second end portion 2041b. Such interface IN may be offset from second hinge axis HA2 in the longitudinal direction, as shown in FIG. 23A. In this regard, interface IN may be closer to second end portion 2041b than second hinge axis HA2, and lever 2004 may extend from interface IN into space 2048 between elongate arms 2047a, 2047b. In the embodiment depicted, lever 2004 extends toward first end portion 2041a beyond hinges 2002a, 2002b. Lever 2004 may be arranged in alignment with hinges 2002a, 2002b such that lever 2004 may be coplanar with hinges 2002a, 2002b. However, in other embodiments, lever 2004 may sit proud of a proximal side of elongate members 2047a, 2047b. Hinges 2002a, 2002b and lever 2004 may be tuned to tailor the amount of line tension needed to overcome the bias of hinges 2002a, 2002b.

Proximal element 2040 may have a first state, a second state, and a third state. In the first state or un-tensioned state, as shown in FIG. 23A, proximal element 2040 is in a lowered position in accordance with a bias of bend feature 2060 to capture tissue. When in the first state, first and second sections 2049a, 2049b align such that they are coaxial or coplanar with longitudinal axis LA. In the second state or tensioned state, as illustrated in FIG. 23B, proximal element line 101, which may be connected to lever 2004, is tensioned so that proximal element 2040 overcomes the bias of bend feature 2060 and is in the raised position. However, in the second state, first and second sections 2049a, 2049b remain aligned as in the first state. Thus, proximal element 2040 has a first configuration in which first and second sections 2049a, 2049b are aligned, and proximal element may be in the first configuration when in the first state and in the second state. The tension of proximal element line 101 for transitioning proximal element 2040 from the first state to the second state may be lower than the tension used to transition proximal element 2040 to the third state.

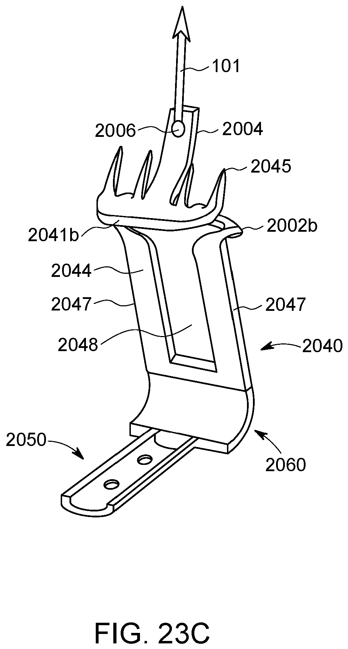

Proximal element 2040 may be transitioned to the third state or high-tension state when it may be desirable to shield frictional elements 2045 from tissue, such as when maneuvering a fixation device, such as fixation device 112, relative to valve leaflets while proximal element 2040 is in the raised position and distal elements 120 are in an open position. To transition proximal element 2040 to the third state, tension in proximal element line 101 is increased relative to that of the second state to overcome the bias of hinges 2002a, 2002b and rotate second section 2049b downwardly or distally about second hinge axis HA2. Thus, proximal element 2040 has a second configuration which it assumes in the third state (i.e., high-tension state). In the second configuration, as shown in FIG. 23C, second section 2049b is angled relative to first section 2049a such that frictional elements 2045 face or point inwardly and/or upwardly toward a center of the fixation to which gripping device 2014 is attached. When the risk of inadvertent tissue engagement is low (e.g., when the fixation device is at the desired position within the valve) and it is time to capture tissue, the excess tension in proximal element line 101 may be released allowing the bias of hinges 2002a, 2002b to return proximal element 2040 to the first configuration and second section 2049b into alignment with first section 2049a.

Gripping device 2014 may be utilized in conjunction with distal elements 120 of fixation device 112, as an example. Additionally, while proximal element 2040 is depicted as being connected to and extending from arm bend feature 2060 and base sections 2050, which are similar to arm bend feature 250 and base section 260 of FIGS. 6A-6C, it should be understood that proximal element 2040 may be connected to and extend from arm bend feature 153 and base section 150 of FIGS. 5A and 5B, for example. Thus, proximal element 2040 may be adapted to couple to a distal element (e.g., distal element 120), such as via base section 2050, or to a center portion of a fixation device via a base section of a gripping device, like in gripping device 114.

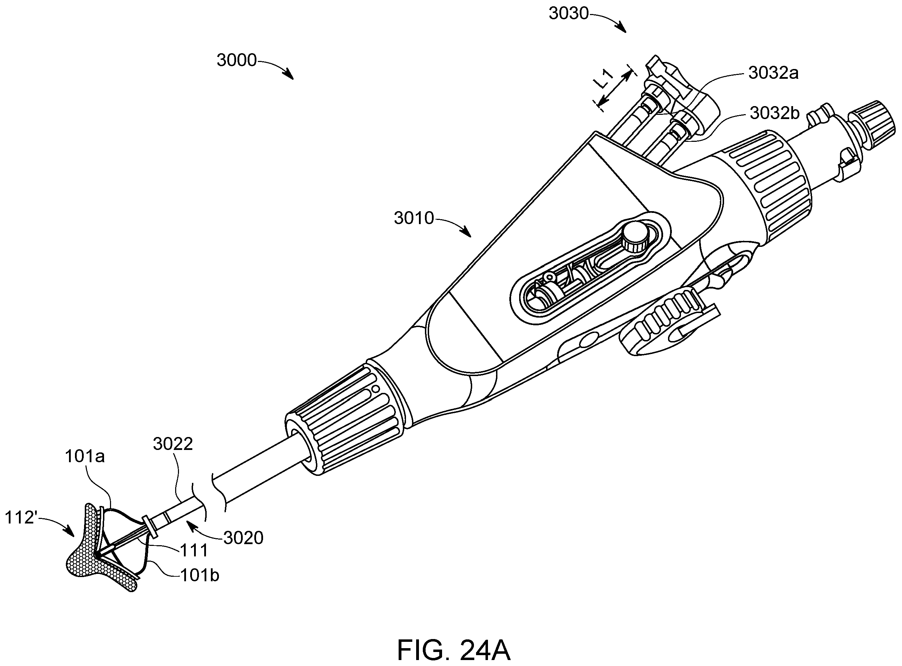

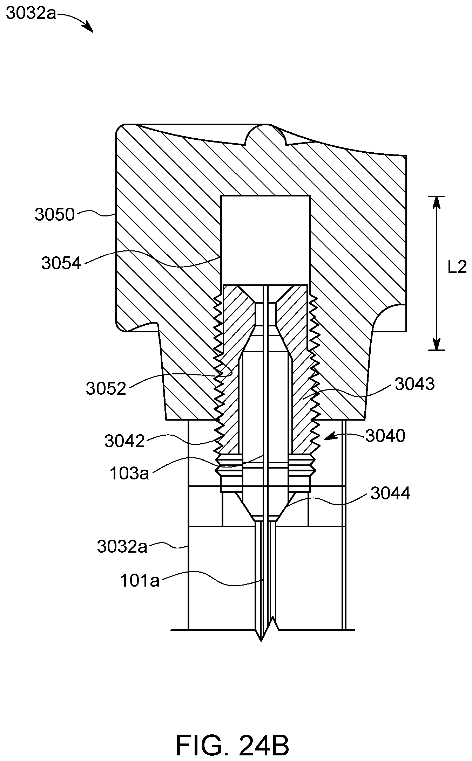

FIGS. 24A and 24B depict a delivery device 3000 according to an embodiment of the present disclosure. Delivery device 3000 may be configured to deliver a fixation device to a target valve. Such fixation device may be fixation device 112' depicted in FIG. 24A which may carry any one of the aforementioned gripping devices 1014, 1114, 1214, and 2014. While it should be understood that any one of the aforementioned gripping devices 1014, 1114, 1214, and 2014 may be included in fixation device 112', the following description is directed to gripping device 1014 for ease of review. Thus, the following description may also be applicable to any one of gripping devices 1114, 1214, and 2014 and their respective proximal elements 1140, 1240, and 2040.

As shown, delivery device 3000 may include a delivery device handle 3010 and a delivery catheter 3020 extending from handle 3010. Delivery catheter 3020 may have a distal end 3022 configured to releasably couple to fixation device 112'. Delivery device handle 3010 may have a plurality of controls for controlling features of fixation device 112'. As shown in FIG. 24A, handle 3010 may include a gripper control assembly 3030 which may be configured to control proximal elements 1040 of fixation device 112'. In this regard, gripper control assembly 3030 may be configured with one or more high-tension features that may be deployed to transition proximal elements 1040 to the high-tensioned state. In particular, gripper control assembly 3030 may include first and second proximal element line handles 3032a, 3032b. First proximal element line handle 3032a may be coupled to a first proximal element line 101a which may correspondingly control or actuate a first proximal element 1040, and a second proximal element line handle 3032b may be coupled with a second proximal element line 101b which may correspondingly control or actuate a second proximal element 1040. First and second proximal element line handles 3032a, 3032b may be releasably coupled to each other such that they may be operated together or independently to move proximal elements 1040 between their respective raised and lowered positions.

FIG. 24B depicts first proximal element line handle 3032a. Second proximal element line handle 3032b may be similarly configured. Therefore, for ease of review, second proximal element line handle 3032b is not discussed separately. First proximal element line handle 3032a may include a connector 3040 and an end cap 3050. Connector 3040 may include a plunger 3043 and a collet 3044. Collet 3044 may be configured to grasp a proximal end portion 103a and/or second end portion 103b of first proximal element line 101a. For example, as shown, a proximal end portion 103a of proximal element line 101a may be secured to collet 3044. As described above, an intermediate portion 103c of first proximal element line 101a may extend from distal end 3022 of delivery catheter 3020 and engage first proximal element 1040, such as to lever 1004 thereof, and distal end portion 103b of proximal element line 101a may be releasably coupled to distal end of delivery catheter 3020, such as to shaft 111 thereof, such as in the manner described with respect to FIGS. 16-17G. Plunger 3043 may be connected to collet 3044 and may have external threads 3042 which may engage internal threads 3052 within handle 3032a and/or end cap 3050.

In operation, first proximal element 1040 may be moved between the first state and second state (i.e., lowered and raised positions) by moving first proximal element line handle 3032a in a proximal-distal direction relative to handle 3010. As shown, such proximal-distal movement may be along a predetermined first length L1 so as to apply a consistent amount of tension on proximal element line 101a to ensure proximal element 1040 does not inadvertently transition to the high-tensioned state. When desired to transition proximal element 1040 to the second configuration (i.e., third state/high-tensioned state), end cap 3050 may be rotated in a first direction relative to first proximal element line handle 3032a which may correspondingly drive plunger 3043 proximally within a cavity 3054 within end cap 3050 to a maximum predetermined second length of L2, as shown in FIG. 24B. Cavity 3054 and the second length L2 defined by cavity 3054 may be configured to apply a consistent amount of additional tension on first proximal element line 101a so as to transition proximal element 1040 to the high-tensioned state but without over tensioning proximal element 1040 and risking failure. When it is desired to transition proximal element 1040 back to the first configuration, end cap 3050 may be rotated in a second direction which may drive plunger 3043 in a distal direction back along second length L2 within cavity 3054 which relieves the high-tension on first proximal element line 101a. From there, first proximal element line handle 3032a may be moved relative to handle 3010 along the first length L1 to lower proximal element 1040 into engagement with tissue.

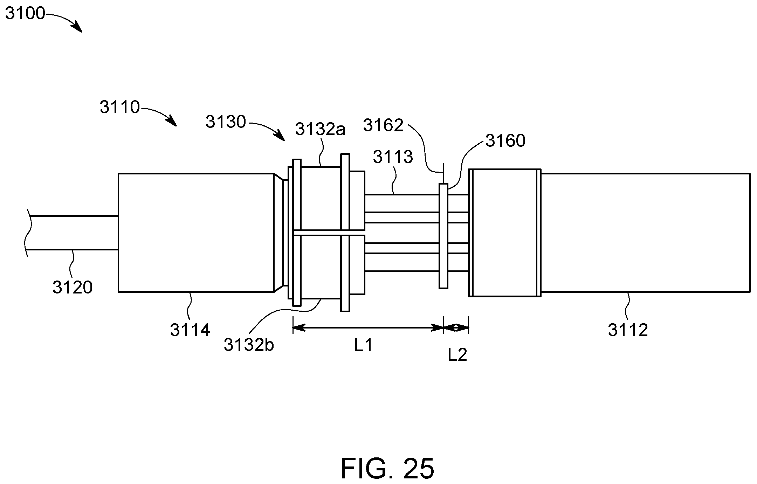

Referring now and in addition to FIG. 25, which depicts a delivery device 3100 according to another embodiment of the present disclosure. Delivery device 3100 includes a delivery device handle 3110 and a delivery catheter 3120 extending from handle 3110. Delivery catheter 3120 may be configured to releasably couple to fixation device 112' as described above. Thus, delivery device 3100 may be configured to releasably coupled to and control fixation device 112' which may carry any one of the aforementioned gripping devices, such as gripping devices 1014, 1114, 1214, and 2014. Again, for ease of review, reference is only made to gripping device 1014.

Delivery catheter handle 3110 may include a first portion or proximal portion 3112, a second portion or distal portion 3114, and an intermediate portion or shaft 3113 extending therebetween. Handle 3110 may also include a gripper control assembly 3130 which may include a first proximal element line handle 3132a and a second proximal element line handle 3132b. First and second proximal element line handles 3132a, 3132b may be respectively coupled to first and second proximal element lines, such as proximal element lines 101a and 101b. First and second proximal element line handles 3132a, 3132b may be slidably connected to shaft 3113 and may be independently moveable to separately and independently actuate proximal elements 1040. However, in some embodiments, proximal element line handles 3132a, 3132b may be releasably coupled for concurrent actuation.

Delivery device handle 3110 may have a high-tension feature. In this regard, each proximal element line handle 3110 may be moveable in a proximal-distal direction along shaft 3113 and along a first length L1 which may move each corresponding proximal element 1040 between their respective lowered and raised positions (i.e., first and second states). Delivery device handle 3110 may also include a restrictor or clip 3160. Restrictor 3160 may be removably coupled to shaft 3113 at first length L1 such that, when restrictor 3160 is coupled to shaft 3113, first and second proximal element line handles 3132a, 3132b are restricted by restrictor 3160 from moving beyond first length L1. However, when it is desired to transition proximal elements 1040 to the high-tension state, restrictor 3160 may be removed from shaft 3113, such as by pulling on a pull tab 3162, for example. Shaft 3113 may have a groove (not shown) which may help secure restrictor 3160 in the desired position on shaft 3113 and may also provide a visual indicator for placing restrictor 3160 back on shaft 3113 and for visually confirming when any one handle 3132a, 3132b has entered a zone of high-tension along shaft 3113. Once restrictor 3160 has been removed, proximal element line handles 3132a, 3132b may be moved an additional distance L2 which may transition proximal elements 1040 to their second configuration (i.e., third state/high-tension state). When desired to return proximal elements 1040 to their first configuration, proximal element line handles 3132a, 3132b may be slid distally to relieve the tension on first and second proximal element lines 101a, 101b.

Although the subject matter disclosed herein has been described with reference to particular embodiments, it is to be understood that these embodiments are merely illustrative of the principles and applications set forth in this disclosure. It is therefore to be understood that numerous modifications may be made to the exemplary embodiments and that other arrangements may be devised, such as combining one or more features of one embodiment with another embodiment or features from a plurality of embodiments, as an example. Thus, the exemplary embodiments herein are not intended to be exhaustive or to limit the disclosed subject matter to such embodiments.

## Claims

1. A fixation device comprising:
a first distal element (120); and
a gripping device (1014, 1114, 1214, 2014) comprising:
a base section (1050, 1150, 1250, 2050),
a first bend feature (1053, 1153, 1253, 2060) defining a first hinge axis (HA1), and
a first proximal element (1040, 1140, 1240, 2040) extending from the first bend feature (1053, 1153, 1253, 2060) and having a first section (1049a, 1149a, 1249a, 2049a), a second section (1049b, 1149b, 1249b, 2049b), and a first hinge (1002, 1102, 1202, 2002a) disposed between the first section and the second section and defining a second hinge axis (HA2), the second section (1049b, 1149b, 1249b, 2049b) having a plurality of frictional elements (1045, 1145, 1245, 2045) extending therefrom and being rotatable about the second hinge axis (HA2) between a first configuration and a second configuration.

2. The fixation device of claim 1, wherein in the first configuration, the first section (1049a, 1149a, 1249a, 2049a) is aligned with the second section (1049b, 1149b, 1249b, 2049b) along a longitudinal axis (LA), and in the second configuration, the second section is angled relative the first section.

3. The fixation device of claim 1 or 2, wherein in the second configuration, the plurality of frictional elements (1045, 1145, 1245, 2045) of the second section (1049b, 1149b, 1249b, 2049b) point in a direction towards the first section.

4. The fixation device of any of the preceding claims, wherein the second section (1049b, 1149b, 1249b, 2049b) includes a distal surface (1044, 1144, 1244, 2044), the distal surface faces the first distal element (120) when the first proximal element (1040, 1140, 1240, 2040) is in the first configuration and faces the first section when in the second configuration.

5. The fixation device as in any of the preceding claims, wherein the first proximal element (1040, 1140, 1240) includes a lever (1004, 1104, 1204) connected to the first hinge (1002, 1102, 1202), the lever extending in a direction toward the first section (1049b, 1149b, 1249b) and being configured to couple to a proximal element line (101).

6. The fixation device of claim 5, wherein the first section (1049b, 1149b) includes a recess (1008, 1108), and the lever (1004, 1104) is disposed within the recess in the first configuration.

7. The fixation device of claim 5, wherein the first section (1249a) includes a proximal surface (1243), and the lever (1204) is disposed above the proximal surface when the first proximal element (1240) is in the first configuration.

8. The fixation device as in any of the preceding claims, wherein the first section (1149a) includes a plurality of frictional elements (1145).

9. The fixation device as in any of the preceding claims, wherein the first proximal element (1040, 1140, 1240, 2040) has an un-tensioned state and a tensioned state, the first proximal element being rotatable about the first hinge axis (HA1) between the un-tensioned state and the tensioned state, the first proximal element (1040, 1140, 1240, 2040) being configured to be in the first configuration in the un-tensioned state and the tensioned state.

10. The fixation device of any of the preceding claims, wherein the first proximal element (1040, 1140, 1240, 2040) has a high-tension state, the first proximal element being in the high tensioned state when in the second configuration.

11. The fixation device of any of the preceding claims, wherein the first proximal element (2040) includes a first elongate member (2047a), a second elongate member (2047b), and a second hinge (2002b), the first hinge (2002a) being on the first elongate member (2047a), and the second hinge (2002b) being on the second elongate member (2047b).

12. The fixation device of claim 11, wherein the first proximal element (2040) includes a first end portion (2041a) and a second end portion (2041b), the first end portion (2041a) being connected to the first bend feature (2060), and the first and second elongate members (2047a, 2047b) extending between the first and second end portions (2041a, 2041b).

13. The fixation device of claim 11 or 12, wherein the first and second elongate members (2047a, 2047b) are offset from each other in a direction transverse to a longitudinal axis (LA) of the first proximal element (2040) so as to form a space (2048) therebetween, the first and second hinges (2002a, 2002b) being aligned such that the first and second hinges together define the second hinge axis (HA2).

14. The fixation device as in one of claims 11-13, wherein the first proximal element (2040) includes a lever (2004) connected to the second end portion (2041b) and extending into the space (2048).

15. The fixation device of claim 14, wherein the lever (2004) and first end portion (2041a) define an interface, the interface being offset in a longitudinal direction relative to the second hinge axis (HA2).
